(19) Europäisches Patentamt
European Patent Office
Office européen des brevets

(11) Numéro de publication : **0 297 995 B1**

# (12) FASCICULE DE BREVET EUROPEEN

(45) Date de publication du fascicule du brevet : **06.03.91 Bulletin 91/10**

(21) Numéro de dépôt : **88401715.3**

(22) Date de dépôt : **01.07.88**

(51) Int. Cl.[5] : **C07C 309/28,** C07C 309/72, C07C 311/15, C07C 317/14, C07C 321/28, C07C 323/00, C07C 311/29, A61K 31/10, A61K 7/48

## (54) Nouveaux dérivés polycycliques aromatiques, leur procédé de préparation et leur utilisation en médecine humaine et vétérinaire et en cosmétique.

(30) Priorité : **03.07.87 FR 8709478**

(43) Date de publication de la demande : **04.01.89 Bulletin 89/01**

(45) Mention de la délivrance du brevet : **06.03.91 Bulletin 91/10**

(84) Etats contractants désignés : **AT BE CH DE ES FR GB GR IT LI NL SE**

(56) Documents cités :
**GB-A- 2 164 644**
**GB-A- 2 164 938**

(73) Titulaire : **L'OREAL**
**14, Rue Royale**
**F-75008 Paris (FR)**

(72) Inventeur : **Maignan, Jean**
**8, rue Halévy**
**F-93290 Tremblay Les Gonesse (FR)**
Inventeur : **Lang, Gérard**
**44, avenue Lacour**
**F-95210 St Gratien (FR)**
Inventeur : **Malle, Gérard**
**18, Grande Rue**
**F-77580 Villiers Sur Morin (FR)**
Inventeur : **Restle, Serge**
**140, rue Anatole France**
**F-93600 Aulnay-Sous-Bois (FR)**

(74) Mandataire : **Stalla-Bourdillon, Bernard et al**
**CABINET NONY & CIE 29, rue Cambacérès**
**F-75008 Paris (FR)**

EP 0 297 995 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux dérivés polycycliques aromatiques, leur procédé de préparation et leur utilisation en médecine humaine et vétérinaire et en cosmétique.

Les composés selon l'invention trouvent une application dans le traitement topique et systémique des affections dermatologiques liées à un désordre de la kératinisation (différenciation-prolifération) et d'affections dermatologiques (ou autres) à composante inflammatoire et/ou immunoallergique et dans le traitement des maladies de dégénerescence du tissu conjonctif, ainsi qu'une activité anti-tumorale.

En outre, ces composés peuvent être utilisés dans le traitement de l'atopie qu'elle soit cutanée ou respiratoire et du psoriasis rhumatoïde.

Ces composés trouvent également une application dans le domaine ophtalmologique notamment dans le traitement des cornéopathies.

Les composés polycycliques aromatiques selon l'invention peuvent être représentés par la formule générale suivante :

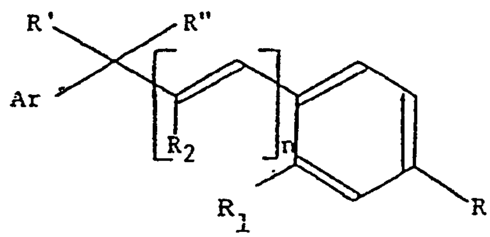

(I)

dans laquelle :
R représente :

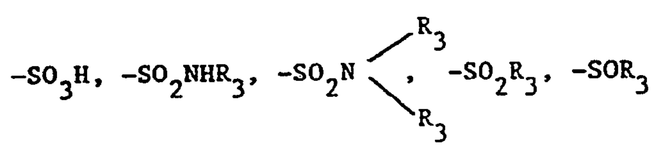

ou $-SR_3$,

$R_3$ représentant un radical alkyle inférieur, linéaire ou ramifié, un radical monohydroxyalkyle, polyhydroxyalkyle, aminoalkyle, carboxyalkyle, aminocarboxyalkyle ou alkoxyalkyle,

R' représente un atome d'hydrogène, un radical alkyle inférieur, un radical alkoxy inférieur, un radical hydroxyle, un radical acyloxy en $C_1$-$C_4$ ou un radical amino,

R" représente un atome d'hydrogène, un radical alkyle inférieur ou un radical alkoxy inférieur, ou

R' et R", pris ensemble, forment un radical oxo (=O), méthano (=$CH_2$) ou hydroxyimino (=N–OH),

$R_1$ représente un atome d'hydrogène ou un radical alkyle inférieur, n est 0 ou 1,

lorsque n=1, $R_2$ représente un atome d'hydrogène ou un radical alkyle inférieur, ou $R_1$ et $R_2$, pris ensemble, forment un radical vinylène (–CH=CH–), et

Ar représente un radical aromatique selon l'une des formules suivantes :

(i)

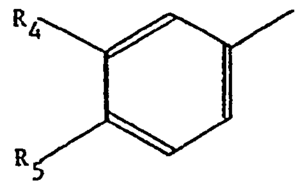

(ii)

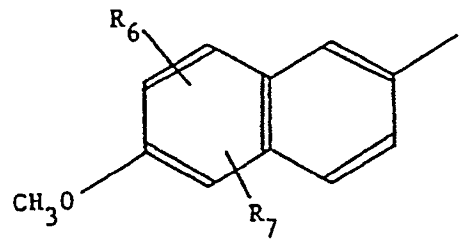

(iii)

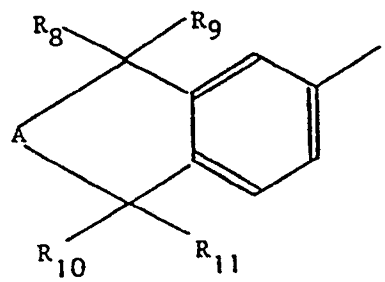

dans lesquelles :

$R_4$, $R_5$, $R_6$, $R_7$, $R_8$, $R_9$, $R_{10}$ et $R_{11}$ représentent un atome d'hydrogène ou un radical alkyle inférieur, au moins un des radicaux $R_4$ et/ou $R_5$ étant différent d'un atome d'hydrogène, et au moins deux des radicaux $R_8$ à $R_{11}$ étant différents d'un atome d'hydrogène,

A représente un radical méthylène ou diméthylène substitué ou non par un radical alkyle inférieur ; lorsque A représente un radical diméthylène, $R_8$ et $R_{10}$ peuvent former ensemble un radical méthylène ou diméthylène,

et les sels desdits composés de formule (I) ainsi que leurs isomères géométriques et optiques.

Par radical alkyle inférieur, linéaire ou ramifié, on doit entendre un radical ayant de 1 à 6 atomes de carbone, en particulier les radicaux méthyle, éthyle, isopropyle, butyle et tertiobutyle.

Par radical monohydroxyalkyle, on doit entendre un radical ayant de 2 à 6 atomes de carbone, notamment un radical hydroxy-2 éthyle ou hydroxy-2 propyle.

Par radical polyhydroxyalkyle, on doit entendre un radical ayant de 3 à 6 atomes de carbone et de 2 à 5 groupes hydroxyles tels que les radicaux dihydroxy-2,3 propyle, dihydroxy-1,3 propyl-2 ou le reste du pentaérythritol.

Par radical aminoalkyle, on doit entendre un radical ayant de 2 à 6 atomes de carbone, tels que les radicaux amino-2 éthyle, amino-2 propyle ou amino-3 propyle.

Par radical carboxyalkyle, on doit entendre un radical ayant de 2 à 7 atomes de carbone, tels que les radicaux carboxyméthyle, carboxy-2 éthyle, carboxy-2 propyle ou carboxy-3 butyle.

Par radical aminocarboxyalkyle, on doit entendre de préférence un radical amino-2 carboxy-2 éthyle ou amino-3 carboxy-3 propyle.

Par radical alkoxy inférieur, on doit entendre un radical ayant de 1 à 6 atomes de carbone tels que les radicaux méthoxy, éthoxy, butoxy ou isopropoxy.

Quand les composés selon l'invention se présentent sous forme de sels, il peut s'agir de sels d'un métal alcalin ou alcalino-terreux ou encore de zinc ou d'une amine organique lorsqu'ils comportent au moins une fonction acide libre, soit de sels d'un acide minéral ou organique notamment de chlorhydrate, de bromhydrate ou de citrate lorsqu'ils comportent au moins une fonction amine.

Parmi les composés particulièrement préférés selon l'invention, on peut notamment citer ceux correspondant à la formule générale suivante :

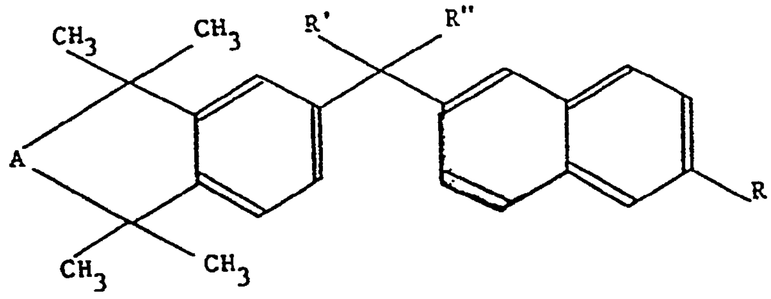

(II)

dans laquelle :

R représente $-SO_2R'_3$, $SO_2NHR'_3$, $SOR'_3$ ou $-SR'_3$,

$R'_3$ représentant un radical alkyle inférieur,

R′ représente un atome d'hydrogène, un radical hydroxyle ou alkyle inférieur,

R″ représente un atome d'hydrogène ou R′ et R″ pris ensemble forment un radical oxo (=0), et

A représente un radical diméthylène ou un radical de formule

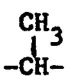 .

Parmi les composés préférés de formule (I) selon l'invention, on peut notamment citer :

- La méthyl[(tétrahydro-5,6,7,8 tétraméthyl-5,5,8,8 naphtyl-2) hydroxyméthyl]-4 phénylsulfone,
- La méthyl[(tétrahydro-5,6,7,8 tétraméthyl-5,5,8,8 naphtyl-2)carbonyl]-4 phénylsulfone,
- Le N-éthyl[(tétrahydro-5,6,7,8 tétraméthyl-5,5,8,8 naphtyl-2) hydroxyméthyl]-4 phénylsulfonamide,
- Le N-éthyl[(tétrahydro-5,6,7,8 tétraméthyl-5,5,8,8 naphtyl-2)carbonyl]-4 phénylsulfonamide,
- La méthyl[E(tétrahydro-5,6,7,8 tétraméthyl-5,5,8,8 naphtyl-2)-3 oxo-3 propényl]-4 phénylsulfone,
- Le méthyl[E(tétrahydro-5,6,7,8 tétraméthyl-5,5,8,8 naphtyl-2)-3 oxo-3 propényl]-4 phénylthioéther,
- Le méthyl[E(tétrahydro-5,6,7,8 tétraméthyl-5,5,8,8 naphtyl-2)-3 oxo-3 propényl]-4 phénylsulfoxyde,
- Le N-éthyl[E(tétrahydro-5,6,7,8 tétraméthyl-5,5,8,8 naphtyl-2)-3 oxo-3 propényl]-4 phénylsulfonamide,
- Le méthyl[E(pentaméthyl-1,1,2,3,3 indanyl-5)-3 oxo-3 propényl]-4 phénylthioéther,
- Le méthyl[E(pentaméthyl-1,1,2,3,3 indanyl-5)-3 oxo-3 propényl]-4 phénylsulfoxyde,
- La méthyl[E(pentaméthyl-1,1,2,3,3 indanyl-5)-3 oxo-3 propényl]-4 phénylsulfone,
- La méthyl[E(pentaméthyl-1,1,2,3,3 indanyl-5)-3 hydroxy-3 propényl]-4 phénylsulfone,
- La méthyl[E(tétrahydro-5,6,7,8 tétraméthyl-5,5,8,8 naphtyl-2)-3 méthoxy-3 propényl]-4 phénylsulfone,
- L'éthyl[(tétrahydro-5,6,7,8 tétraméthyl-5,5,8,8 naphtyl-2)carbonyl]-6 naphtylsulfone-2,
- L'éthyl[(pentaméthyl-1,1,2,3,3 indanyl-5)carbonyl]-6- naphtylsulfone-2,
- L'éthyl[E(tétraméthyl-1,1,3,3 indanyl-5)-3 oxo-3 propényl]-4 phénylsulfone,
- La méthyl[E(pentaméthyl-1,1,2,3,3 indanyl-5)-3 méthoxy-3 propényl]-4 phénylsulfone,
- L'éthyl[(tétraméthyl-1,1,3,3 indanyl-5)carbonyl]-6 naphtylsulfone-2,
- La dihydroxy-2′,3′ propyl[(tétrahydro-5,6,7,8 tétraméthyl-5,5,8,8 naphtyl-2)carbonyl]-6 naphtylsulfone-2,
- L'éthyl[(tétrahydro-5,6,7,8 tétraméthyl-5,5,8,8 naphtyl-2) hydroxyméthyl]-6 naphtylsulfone-2,
- L'éthyl[(méthano-5,8 tétrahydro-5,6,7,8 naphtyl-2)carbonyl]-6 naphtylsulfoxyde-2,
- L'éthyl[(méthano-5,8 tétrahydro-5,6,7,8 naphtyl-2) carbonyl]-6 naphtylsulfone-2
- L'éthyl[(tétrahydro-5,6,7,8 tétraméthyl-5,5,8,8 naphtyl-2)méthylène]-6 naphtylsulfone-2,
- L'éthyl[(tétrahydro-5,6,7,8 tétraméthyl-5,5,8,8 naphtyl-2)-1 hydroxy-1 éthyl]naphtylsulfone-2,
- Le N-éthyl[(tétrahydro-5,6,7,8 tétraméthyl-5,5,8,8 naphtyl-2)carbonyl]-4 naphtylsulfonamide-2,
- L'éthyl[(tétrahydro-5,6,7,8 tétraméthyl-5,5,8,8 naphtyl-2)-1 hydroxy-1 éthyl]-6 naphtyl thioéther-2,
- L'éthyl[(tétrahydro-5,6,7,8 tétraméthyl-5,5,8,8 naphtyl-2)-1 éthényl]-6 naphtyl thioéther-2,
- L'éthyl[(tétrahydro-5,6,7,8 tétraméthyl-5,5,8,8, naphtyl-2)carbonyl]-6 naphtylsulfoxyde-2,
- L'éthyl[(diméthyl-5,8 méthoxy-6 naphtyl-2)carbonyl]-6 naphtylsulfone-2,
- L'éthyl[(diméthyl-5,8 méthoxy-6 naphtyl-2)hydroxyméthyl]-6 naphtylsulfone-2, et

– L'éthyl[(diméthyl-5,8 méthoxy-6 naphtyl-2)méthylène]-6 naphtylsulfone-2.

La présente invention a également pour objet le procédé de préparation des composés de formule (I).

Ces composés peuvent être obtenus selon différentes méthodes en fonction de leurs structures.

Selon un premier mode de réalisation, les composés selon l'invention sont obtenus selon le schéma réactionnel suivant :

Schéma A

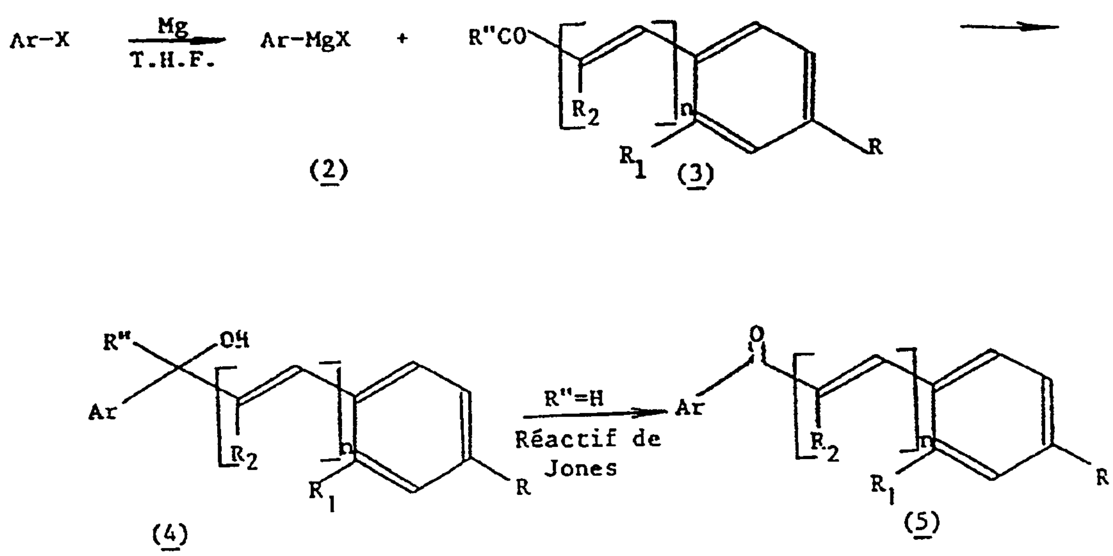


(2) (3)

(4) (5)

X = Br ou Cl

R″ = H ou Alkyle

Ce procédé consiste à préparer, dans un premier temps, un dérivé organomagnésien (2) à partir d'un composé halogéné aromatique (1) selon la méthode classique de préparation des réactifs de Grignard.

Celui-ci est ensuite mis à réagir avec un aldéhyde de formule (3) (R″=H) ou une cétone de formule (3) (R″=alkyle inférieur) dans un solvant organique tel que le tétrahydrofuranne (T.H.F.) à une température comprise entre –10°C et +20°C mais de préférence d'environ 0°C.

Lorsque le composé de formule (3) est un aldéhyde, l'alcool secondaire obtenu (4) (R″=H) peut être oxydé en composé carbonylé correspondant (5) en utilisant comme agent oxydant le réactif de Jones.

Les composés halogénés aromatiques (1) sont de préférence des dérivés bromés et parmi ceux-ci, on peut citer :

– le bromo-2 tétrahydro-5,6,7,8 tétraméthyl-5,5,8,8, naphtalène,

– le bromo-5 tétraméthyl-1,1,3,3 indane,

– le bromo-5 pentaméthyl-1,1,2,3,3 indane,

– le bromo-2 méthano-5,8 tétrahydro-5,6,7,8 naphtalène, et

– le bromo-2 diméthyl-5,8 méthoxy-6 naphtalène.

Ces dérivés bromés sont obtenus par action du brome sur, respectivement :

– le tétrahydro-1,2,3,4 tétraméthyl-1,1,4,4 naphtalène préparé selon la méthode décrite dans J. Am. Chem. Soc., 63, 36-44, (1940),

– le tétraméthyl-1,1,3,3 indane, et

– le pentaméthyl-1,1,2,3,3 indane préparés selon le mode opératoire décrit dans le brevet français n°1.392.804,

– Le méthano-1,4 tétrahydro-1,2,3,4 naphtalène ou benzonorbornène préparé selon la méthode décrite dans J. Org. Chem., 32, 893-901, (1967),

– Le diméthyl-5,8 méthoxy-6 naphtalène préparé suivant la méthode décrite par M. FETIZON et N.T. ANH, Bull. Soc. Chim. Fr., 3028, (1965).

Les aldéhydes ou cétones de formule (3) sont des produits du commerce ou peuvent être obtenus selon les méthodes connues.

Lorsque dans les composés de formule (I) n=0 ou n=1 et $R_1$ et $R_2$, pris ensemble, forment un radical vinylidène (cycle naphtalénique), ceux-ci sont de préférence obtenus selon le schéma réactionnel suivant:

Schéma B

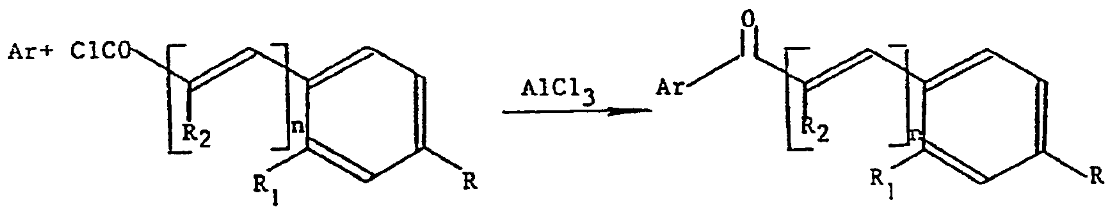


(6) (7) (5)

Cette méthode consiste à acyler dans les conditions habituelles de la réaction de Friedel-Crafts un composé aromatique (6) par un chlorure d'acide benzénique ou naphtalénique de formule (7). Les chlorures d'acide de formule (7) sont facilement accessibles à partir des thioéthers benzéniques ou naphtaléniques.

Lorsque dans les composés de formule (I) n=1 et $R_1$ et $R_2$ représentent un atome d'hydrogène ou un radical alkyle inférieur, ceux-ci sont obtenus de préférence selon le schéma réactionnel suivant :

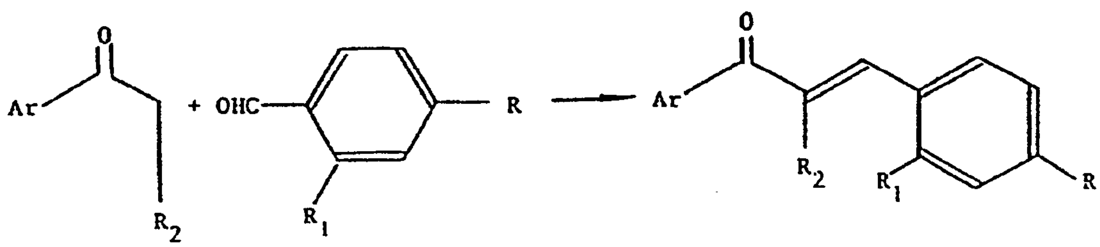

(8) (9) (10)

Cette méthode consiste à condenser dans les conditions de la réaction de Claisen-Schmidt un aldéhyde benzénique (9) sur une cétone aromatique de formule (8). De préférence, la réaction est effectuée en présence de soude ou de potasse dans un solvant organique tel que l'éthanol. Cette méthode permet d'accéder aux cétones insaturées de configuration "E" avec de bons rendements. La nature du radical R doit être compatible avec ce type de condensation, elle convient lorsque R est un radical thioéther, sulfoxyde, sulfone ou sulfonamide.

A partir des composés carbonylés (5) et (10), on peut accéder aux autres composés selon l'invention.

Ainsi, les composés dans lesquels R'=OH et R"=H sont obtenus par réduction en présence de borohydrure de sodium dans un solvant organique tel que l'éthanol ou le T.H.F..

Les composés dans lesquels R'=R"=H sont obtenus par réduction au zinc des dérivés cétoniques (R' et R"=oxo) dans l'acide acétique en présence d'acide chlorhydrique.

Ces réactions de réduction doivent bien entendu être compatibles avec la nature du radical R mais elles ne soulèvent aucune difficulté lorsque $R=SO_2R_3$.

Les composés dans lesquels R'=acyloxy et R"=H sont obtenus en faisant réagir une forme activée d'acide tel qu'un anhydride ou un chlorure d'acide sur un composé de formule (I) dans laquelle R'=OH et R"=H.

Les composés dans lesquels R'=alkoxy et R"=H sont de même obtenus à partir des composés de formule (I) dans laquelle R'=OH et R"=H selon les méthodes connues.

Les composés de formule (I) dans laquelle R' et R"=méthano ($CH_2$=) sont obtenus par réaction de Wittig selon le schéma réactionnel suivant :

Schéma D

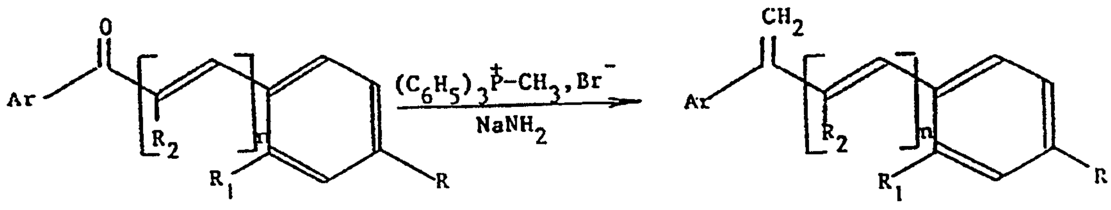


Les composés de formule (I) dans laquelle R′ et R″=hydroxyimino (=N–OH) sont obtenus par action de l'hydroxylamine sur les composés carbonylés correspondants.

La présente invention a également pour objet, à titre de médicament, les composés de formule (I) telle que définie ci-dessus.

Ces composés, du fait de la présence d'une substitution par une fonction thioéther ou ses dérivés d'oxydation, sont moins toxiques que les dérivés correspondants substitués par une fonction carboxylique.

Ces composés conviennent particulièrement bien pour traiter les affections dermatologiques liées à un désordre de la kératinisation (différenciation-prolifération) ainsi que les affections dermatologiques, ou autres, à composante inflammatoire et/ou immunoallergique notamment :

- les acnés vulgaires, comédoniennes ou polymorphes, les acnés séniles, solaires, et les acnés médicamenteuses ou professionnelles,
- les formes étendues et/ou sévères de psoriasis, et les autres troubles de la kératinisation, et notamment les ichtyoses et états ichtyosiformes
- la maladie de Darier,
- les kératodermies palmo-plantaires,
- les leucoplasies et états leucoplasiformes, le lichen plan
- toutes proliférations dermatologiques bénignes ou malignes, sévères ou étendues.

Ils sont également actifs dans le traitement des tumeurs, du psoriasis rhumatoïde, des atopies cutanées ou respiratoires ainsi que de certains problèmes ophtalmologiques relatifs aux cornéopathies.

La présente invention a donc également pour objet des compositions médicamenteuses contenant au moins un composé de formule (I) tel que défini ci-dessus, ou un de ses sels ou un de ses isomères.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels et/ou un de ses isomères.

Les composés selon l'invention sont généralement administrés à une dose journalière d'environ 0,01 mg/Kg à 5 mg/Kg de poids corporel.

Comme support des compositions, on peut utiliser tout support conventionnel, le composé actif se trouvant soit à l'état dissous, soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base des composés selon l'invention se présentent sous forme d'onguents, de teintures, de crèmes, de pommades, de poudres, de timbres, de tampons imbibés, de solutions, de lotions, de gels, de sprays ou encore de suspensions.

Ces compositions par voie topique peuvent se présenter soit sous forme anhydre, soit sous forme aqueuse selon l'indication clinique.

Par voie oculaire, ce sont principalement des collyres.

Les compositions par voie topique ou oculaire contiennent de 0,0001 à environ 5% d'au moins un composé de formule (I) telle que définie ci-dessus et de préférence de 0,001 à 1% en poids par rapport au poids total de la composition.

Les composés de formule (I), selon l'invention, trouvent également une application dans le domaine cosmétique, en particulier dans l'hygiène corporelle et capillaire et notamment pour le traitement des peaux

à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels et/ou un de ses isomères, cette composition se présentant notamment sous forme de lotion, gel, savon, shampooing ou crème.

La concentration en composé(s) de formule (I), dans les compositions cosmétiques, est comprise entre 0,0001 et 2% en poids et de préférence entre 0,001 et 1% en poids.

Les compositions médicamenteuses et cosmétiques selon l'invention peuvent contenir des additifs inertes ou même pharmacodynamiquement ou cosmétiquement actifs et notamment : des agents hydratants comme la thiamorpholinone et ses dérivés ou l'urée ; des agents antiséborrhéiques ou antiacnéiques, tels que la S-carboxyméthylcystéine, la S-benzyl-cystéamine, leurs sels et leurs dérivés, la tioxolone ou le peroxyde de benzoyle ; des antibiotiques comme l'érythromycine et ses esters, la néomycine ou les tétracyclines et les polyméthylène-4,5 isothiazolones-3 ; des agents favorisant la repousse des cheveux comme le "Minoxidil" (diamino-2,4-pipéridino-6-pyrimidine oxyde-3) et ses dérivés, le Diazoxide (chloro-7 méthyl-3 benzothiadiazine-1,2,4 dioxyde-1,1) et le Phénytoïn (diphényl-5,5 imidazolidine dione-2,4) ; des agents anti-inflammatoires stéroïdiens et non stéroïdiens ; des caroténoïdes et notamment le β -carotène ; des agents anti-psoriasiques tels que l'anthraline et ses dérivés et les acides eicosatétraynoïque-5,8,11,14 et triynoïque-5,8,11, leurs esters et leurs amides.

Les compositions selon l'invention peuvent également contenir des agents d'amélioration de la saveur, des agents conservateurs, des agents stabilisants, des agents régulateurs d'humidité, des agents régulateurs de pH, des agents émulsionnants, des filtres UV-A et UV-B, des anti-oxydants tels que l'α-tocophérol, le butylhydroxy-anisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

Les exemples A à E concernent la préparation des intermédiaires de synthèse pour obtenir les composés de formule générale I.

Toutes les structures décrites dans ces différents exemples sont confirmées en $^{1}H$ R.M.N. 80MHz et dans certains cas par $^{13}C$ R.M.N. 100MHz.

Dans les exemples de préparation des composés, l'évolution des milieux réactionnels est toujours suivie en chromatographie couche mince (C.C.M). Les temps de réaction indiqués correspondent donc à la majorité du produit de départ transformé.

## EXEMPLE A

### Préparation du méthylsulfonyl-4 benzaldéhyde et du méthylsulfinyl-4 benzaldéhyde

A une solution agitée de 30 g de méthylthio-4 benzaldéhyde dans 250 cm³ d'acide formique, on ajoute lentement à température ambiante 44 cm³ d'eau oxygénée à 30% (2 équivalents). La réaction est exothermique et la température s'élève jusqu'à 75°C. Lorsque le produit de départ est totalement transformé, la solution obtenue est concentrée à environ 50 cm³ puis versée dans l'eau. Le mélange est alors neutralisé par addition de soude 6N, on extrait trois fois par 200 cm³ d'acétate d'éthyle et les phases organiques, rassemblées, sont lavées par une solution aqueuse d'hydrosulfite de sodium puis à l'eau et enfin séchées sur sulfate de magnésium. Le solvant est éliminé par évaporation sous vide. Le solide obtenu est dissous dans le toluène et la solution déposé sur une colonne de chromatographie de gel de silice. En utilisant comme éluant le mélange acétate d'éthyle-heptane (7-3), puis après évaporation de la phase éluante, on isole 16 g de méthylsulfonyl-4 benzaldéhyde et 7 g de méthylsulfinyl-4 benzaldéhyde.

## EXEMPLE B

### Préparation du N-éthyl formyl-4 phénylsulfonamide

Une suspension de 26 g de paratoluène sulfochlorure dans 200 cm³ d'eau est refroidie à 0°C. Sous agitation, on ajoute goutte à goutte 100 cm³ d'une solution aqueuse d'éthylamine à 40% dans l'eau. A la fin de l'addition le milieu réactionnel est encore agité deux heures à 0°C puis deux heures à température ambiante. Le solide en suspension est filtré, lavé plusieurs fois à l'eau puis séché sous pression réduite. On obtient 24 g de N-éthyl(méthyl-4 phényl) sulfonamide.

A une solution de 15 g de N-éthyl(méthyl-4 phényl) sulfonamide dans 75 $cm^3$ d'anhydride acétique on ajoute lentement, à température ambiante, 18 $cm^3$ d'acide sulfurique concentré.

A cette solution refroidie à 0°C, on ajoute très lentement une solution de 21 g d'oxyde de chrome dissous dans 90 $cm^3$ d'anhydride acétique. L'addition terminée, le mélange est maintenu une heure à 0°C, puis il est abandonné une nuit à température ambiante. Le lendemain, le mélange est versé sur 1 litre d'eau glacée et extrait trois fois par 200 $cm^3$ d'acétate d'éthyle. Les phases organiques, rassemblées, sont lavées par une solution aqueuse de bicarbonate de sodium jusqu'à pH 6 des eaux de lavage. La solution d'acétate d'éthyle est séchée sur sulfate de magnésium et concentrée. Le liquide visqueux obtenu est alors agité pendant trois jours à 50°C dans 1 litre d'acide chlorhydrique N. Cette solution acide est extraite trois fois par 150 $cm^3$ d'acétate d'éthyle. Les phases d'acétate d'éthyle sont lavées par une solution aqueuse saturée de chlorure d'ammonium puis à l'eau et enfin séchée sur sulfate de magnésium.

Par évaporation sous vide de l'acétate d'éthyle, on obtient 7 g de N-éthyl(formyl-4 phényl)sulfonamide.

## EXEMPLE C

### Préparation de l'acétyl-2 éthylthio-6 naphtalène et de sa sulfone

#### 1) Ethylthio-2 naphtalène

A une solution agitée sous atmosphère inerte, à température ambiante de 40 g de mercapto-2 naphtalène dans 1 litre d'éthanol anhydre, on ajoute lentement 24,4 g de potasse. L'addition terminée, l'agitation est maintenue 2 heures, puis on ajoute alors goutte à goutte une solution de 21 $cm^3$ de bromoéthane dissous dans 100 $cm^3$ d'éthanol anhydre. Après 3 heures d'agitation, le milieu réactionnel est abandonné une nuit puis filtré et le filtrat est concentré sous vide. Le produit brut obtenu est agité dans 800 $cm^3$ d'eau puis extrait 4 fois par 150 $cm^3$ de dichlorométhane. Les phases de dichlorométhane sont rassemblées, lavées avec une solution saturée de chlorure d'ammonium puis séchées sur sulfate de magnésium. Le solvant est rectifié sous vide et on obtient 38 g d'éthylthio-2 naphtalène utilisé tel quel pour les réactions suivantes.

#### 2) Acétyl-2 éthylthio-6 naphtalène

A un mélange agité à l'abri de l'humidité de l'air de 10 g d'éthylthio-2 naphtalène et de 4,5 $cm^3$ de chlorure d'acétyle dans 200 $cm^3$ de dichloro-1,2 éthane, on ajoute par petites portions 11 g de chlorure d'aluminium de façon à maintenir la température à 30°C.

A la fin de l'addition le milieu réactionnel est encore agité pendant 3 heures à température ambiante puis versé dans 200 $cm^3$ d'une solution aqueuse saturée de chlorure d'ammonium. Le mélange est extrait trois fois par 100 $cm^3$ de dichlorométhane et les phases organiques rassemblées sont lavées au bicarbonate de sodium puis séchées sur sulfate de magnésium et concentrées.

On obtient 7 g d'acétyl-2 éthylthio-6 naphtalène de point de fusion : 69°C.

#### 3) Acétyl-2 éthylsulfonyl-6 naphtalène

A une solution de 7 g d'acétyl-2 éthylthio-6 naphtalène dans 200 $cm^3$ de dichlorométhane agitée à 0°C, on ajoute par petites quantités 14 g d'acide métachloroperbenzoïque. L'addition terminée, on maintient l'agitation du milieu réactionnel pendant 3 heures à 0°C et ensuite pendant 2 heures à température ambiante. Le mélange est alors versé dans 300 $cm^3$ d'une solution aqueuse saturée de chlorure d'ammonium puis on extrait trois fois par 100 $cm^3$ de dichlorométhane. Les phases organiques rassemblées sont alors lavées par une solution diluée de bicarbonate de sodium, séchées sur sulfate de magnésium et concentrées. Le solide obtenu est alors lavé avec un mélange d'heptane-toluène (10-5) porté à 50°C. Après séchage, on obtient 7 g d'acétyl-2 éthylsulfonyl-6 naphtalène sous forme de cristaux jaunes de point de fusion : 133°C.

## EXEMPLE D

### Préparation du chlorure de l'acide éthylsulfonyl-6 naphtalène carboxylique-2

#### 1) Acide éthylsulfonyl-6 naphtalène carboxylique-2

On prépare dans un premier temps une solution d'hypobromite de sodium en ajoutant, à 0°C, 3 $cm^3$ de brome à une solution agitée contenant 7,5 g de soude dans 35 $cm^3$ d'eau. Ensuite, on introduit lentement

une suspension de 1 g d'acétyl-2 éthylsulfonyl-6 naphtalène dispersée dans 20 cm³ de dioxanne de façon à maintenir la température du milieu réactionnel inférieure à 5°C. A la fin de l'addition, cette température est encore maintenue pendant une heure et ensuite on ajoute lentement à température ambiante 15 g de métabisulfite de sodium dissous dans 150 cm³ d'eau. Par acidification du mélange à l'acide chlorhydrique, l'acide attendu précipite, il est filtré, lavé abondamment à l'eau et séché. On obtient 0,95 g d'acide éthylsulfonyl-6 naphtalène carboxylique-2 sous forme d'une poudre blanche de point de fusion : 241°C.

2) Chlorure de l'acide éthylsulfonyl-6 naphtalène carboxylique-2

Une suspension de 1 g de l'acide précédent dans 30 cm³ de chlorure de thionyle est portée à 60°C pendant 1 heure. L'acide de départ se solubilise progressivement. Le chlorure de thionyle est alors éliminé par évaporation sous vide et le solide obtenu est lavé à l'hexane. On obtient 1 g de chlorure de l'acide éthylsulfonyl-6 naphtalène carboxylique-2 sous forme d'une poudre brune qui est utilisé directement pour les réactions de Friedel et Crafts.

EXEMPLE E

Préparation de l'acétyl-5 pentaméthyl-1,1,2,3,3 indane

A un mélange agité à température ambiante à l'abri de l'humidité de l'air de 25 g de pentaméthyl-1,1,2,3,3 indane et de 13 cm³ de chlorure d'acétyle, on ajoute par petites quantités 23 g de chlorure d'aluminium de façon à ce que la température ne dépasse pas 40°C. L'agitation est maintenue pendant 2 heures après la fin de l'addition et le milieu réactionnel est abandonné pendant une nuit. Le lendemain, on verse dans 200 cm³ d'eau glacée puis extrait trois fois par 150 cm³ de dichlorométhane. Les phases organiques sont rassemblées, lavées au bicarbonate de sodium puis à l'eau et séchées sur sulfate de magnésium. Le solvant est éliminé par évaporation sous vide et on obtient 31 g d'acétyl-5 pentaméthyl-1,1,2,3,3 indane sous forme liquide qui est utilisé directement pour les réactions suivantes.

EXEMPLE I

Préparation de la méthyl[(tétrahydro-5,6,7,8 tétraméthyl-5,5,8,8 naphtyl-2)hydroxyméthyl]-4 phényl sulfone

A une solution agitée à l'abri de l'humidité de l'air de 5 g de bromo-2 tétrahydro-5,6,7,8 tétraméthyl-5,5,8,8 naphtalène dans 30 cm³ de tétrahydrofuranne anhydre, on ajoute en une fois 0,5 g de magnésium. La réaction est amorcée par chauffage d'un endroit très localisé du mélange réactionnel lorsque l'agitation est stoppée. Lorsque le solvant atteint sa température d'ébullition, le mélange est dilué par addition de 80 cm³ de T.H.F. puis sous agitation le mélange est chauffé au reflux pendant une heure, temps au bout duquel le magnésium est transformé. La solution obtenue est alors refroidie à 0°C et on ajoute goutte à goutte une solution de 1,72 g de méthylsulfonyl-4 benzaldéhyde obtenu à l'exemple A dissous dans 80 cm³ de T.H.F.. Le milieu réactionnel se colore en jaune, l'agitation est maintenue encore une heure à 0°C puis le mélange réactionnel est abandonné une nuit à température ambiante. Le lendemain, on verse dans 200 cm³ d'une solution saturée de chlorure d'ammonium. Le mélange obtenu est alors extrait trois fois à l'aide de 150 cm³ de diéthyléther et les phases organiques, rassemblées, sont lavées avec 200 cm³ d'eau puis séchées sur sulfate de magnésium et concentrées. On obtient 5 g d'un liquide jaune que l'on cristallise dans l'hexane à –25°C. On essore et on obtient après séchage 3,5 g de cristaux que l'on recristallise dans un mélange toluène-hexane.

On obtient 2 g de méthyl[(tétrahydro-5,6,7,8 tétraméthyl-5,5,8,8 naphtyl-2)hydroxyméthyl]-4 phénylsulfone ainsi obtenus se présentent sous forme de cristaux blancs de point de fusion : 134°C.

Analyse élémentaire : $C_{22}H_{28}O_3S$

| | C | H | O | S |
|---|---|---|---|---|
| Calculé | 70,93 | 7,57 | 12,89 | 8,61 |
| Trouvé | 71,03 | 7,63 | 13,01 | 8,30 |

EXEMPLE II

Préparation de la méthyl[(tétrahydro-5,6,7,8 tétraméthyl-5,5,8,8 naphtyl-2)carbonyl]-4 phényl sulfone

A une solution agitée à température ambiante de 2,5 g de méthyl[(tétrahydro-5,6,7,8 tétraméthyl-5,5,8,8 naphtyl-2)hydroxyméthyl]-4 phényl sulfone obtenu à l'exemple I dans 100 cm³ d'acétone, on ajoute goutte à goutte le réactif de Jones préparé au préalable en ajoutant 1,9 cm³ d'acide sulfurique concentré à une solution de 2,2 g de bichromate de potassium solubilisé dans 15 cm³ d'eau. A la fin de l'addition, le mélange est encore agité pendant deux heures puis abandonné une nuit à température ambiante.

L'acétone est ensuite éliminée par évaporation sous vide et le mélange obtenu est repris par 200 cm³ d'eau et extrait trois fois à l'aide de 100 cm³ de diéthyléther. Les phases organiques rassemblées sont lavées avec une solution aqueuse de bicarbonate de sodium, puis à l'eau et enfin séchées sur sulfate de magnésium.

Après évaporation du solvant, on obtient 2 g d'une poudre blanche que l'on solubilise dans le minimum de toluène. La solution est déposée sur une colonne de gel de silice et le produit attendu élué au mélange hexane-acétate d'éthyle (4-2). Après évaporation de l'éluant, on obtient 1,5 g de la méthyl[(tétrahydro-5,6,7,8 tétraméthyl-5,5,8,8 naphtyl-2)carbonyl]-4 phényl sulfone sous forme d'une poudre blanche de point de fusion : 161°C.

Analyse élémentaire : $C_{22}H_{26}O_3S$

| | C | H | O | S |
|---|---|---|---|---|
| Calculé | 71,31 | 7,07 | 12,96 | 8,65 |
| Trouvé | 70,91 | 7,19 | 13,24 | 8,55 |

EXEMPLE III

Préparation du N-éthyl[(tétrahydro-5,6,7,8 tétraméthyl-5,5,8,8 naphtyl-2)hydroxyméthyl]-4 phényl sulfonamide

Suivant le mode opératoire décrit à l'exemple I, on transforme en organomagnésien correspondant 2,5 g de bromo-2 tétrahydro-5,6,7,8 tétraméthyl-5,5,8,8 naphtalène solubilisé dans 30 cm³ de T.H.F. par addition de 0,27 g de magnésium.

A cette solution refroidie à 0°C, on ajoute goutte à goutte une solution de 1 g de N-éthyl formyl-4 phényl sulfonamide (obtenu à l'exemple B) solubilisé dans 20 cm³ de T.H.F..

Le mélange est agité 2 heures à température ambiante puis abandonné une nuit. Il est alors versé sur 100 cm³ d'une solution aqueuse saturée de chlorure d'ammonium puis extrait trois fois par 100 cm³ de diéthyléther.

Les phases organiques rassemblées sont lavées à l'eau, séchées sur sulfate de magnésium et concentrées sous pression réduite. Le produit brut est solubilisé dans le minimum de toluène et déposé sur une colonne de chromatographie de gel de silice. Après élution au mélange hexane-acétate d'éthyle (1-1) et concentration de l'éluant, le produit attendu cristallise dans un mélange hexane-éther isopropylique. On obtient ainsi 0,8 g de N-éthyl [(tétrahydro-5,6,7,8 tétraméthyl-5,5,8,8 naphtyl-2)hydroxyméthyl]-4 phényl sulfonamide sous forme de cristaux blancs de point de fusion : 175°C.

Analyse élémentaire : $C_{23}H_{31}NO_3S$

| | C | H | N | O | S |
|---|---|---|---|---|---|
| Calculé | 68,79 | 7,78 | 3,49 | 11,95 | 7,99 |
| Trouvé | 68,76 | 7,76 | 3,35 | 11,87 | 7,94 |

Au cours de cette réaction, l'alcool secondaire obtenu s'oxyde partiellement en dérivé carbonylé correspondant décrit à l'exemple IV.

EXEMPLE IV

Préparation du N-éthyl[(tétrahydro-5,6,7,8 tétraméthyl-5,5,8,8 naphtyl-2)carbonyl]-4 phényl sulfonamide

A une solution agitée à température ambiante de 2 g de N-éthyl [(tétrahydro-5,6,7,8 tétraméthyl-5,5,8,8 naphtyl-2)hydroxyméthyl]-4 phényl sulfonamide préparé à l'exemple III dans 100 $cm^3$ d'acétone, on ajoute le réactif de Jones préparé selon l'exemple II. Le milieu réactionnel est ensuite traité dans les mêmes conditions. Le dérivé carbonylé est purifié par passage sur chromatographie de gel de silice. Il est élué au mélange hexane-acétate d'éthyle (4-1) et recristallisé dans un mélange hexane-éther isopropylique. On obtient 1,5 g de N-éthyl [(tétrahydro-5,6,7,8 tétraméthyl-5,5,8,8 naphtyl-2)carbonyl]-4 phényl sulfonamide.

C'est un solide blanc de point de fusion : 134°C.

Analyse élémentaire : $C_{23}H_{29}NO_3S$

| | C | H | N | O | S |
|---|---|---|---|---|---|
| Calculé | 69,14 | 7,32 | 3,51 | 12,01 | 8,03 |
| Trouvé | 69,28 | 7,34 | 3,42 | 12,05 | 7,94 |

EXEMPLE V

Préparation de la méthyl[E(tétrahydro-5,6,7,8 tétraméthyl-5,5,8,8 naphtyl-2)-3 oxo-3 propényl]-4 phényl sulfone

A une solution de 2,3 g de potasse dans un mélange de 80 $cm^3$ d'eau et 30 $cm^3$ d'éthanol agitée à l'abri de la lumière et à température ambiante, on ajoute rapidement une suspension contenant 4,6 g d'acétyl-2 tétrahydro-5,6,7,8 tétraméthyl-5,5,8,8 naphtalène et 3,7 g de méthyl formyl-4 phényl sulfone dans 70 $cm^3$ d'éthanol.

L'agitation est maintenue pendant 3 heures et le milieu est abandonné ensuite 2 jours à température ambiante temps au bout duquel la formyl-4 phénylsulfone est totalement transformée. L'éthanol est alors éliminé par évaporation sous vide et la suspension obtenue est reprise par 200 $cm^3$ d'eau puis extraite trois fois par 150 $cm^3$ d'acétate d'éthyle. Les phases d'acétate d'éthyle sont rassemblées, lavées avec une solution aqueuse saturée de chlorure d'ammonium puis à l'eau et séchées sur sulfate de magnésium.

Après évaporation sous pression réduite de l'acétate d'éthyle, les 6,5 g de poudre jaune obtenus sont recristallisés dans un mélange toluène-hexane. On obtient 3,5 g de méthyl[E(tétrahydro-5,6,7,8 tétraméthyl-5,5,8,8 naphtyl-2)-3 oxo-3 propényl]-4 phényl sulfone sous forme de cristaux jaune clair de point de fusion : 135°C.

Analyse élémentaire : $C_{24}H_{28}O_3S$

| | C | H | O | S |
|---|---|---|---|---|
| Calculé | 72,69 | 7,12 | 12,10 | 8,09 |
| Trouvé | 72,87 | 7,07 | 12,39 | 7,90 |

EXEMPLE VI

Préparation du méthyl[E(tétrahydro-5,6,7,8 tétraméthyl-5,5,8,8 naphtyl-2)-3 oxo-3 propényl]-4 phényl thioéther

On ajoute à l'abri de la lumière et à température ambiante une solution de 6,5 g de méthylthio-3 benzaldéhyde et de 10 g d'acétyl-2 tétrahydro-5,6,7,8 tétraméthyl-5,5,8,8 naphtalène dans 140 $cm^3$ d'éthanol à une solution agitée de 5,8 g de potasse dissoute dans un mélange de 160 $cm^3$ d'eau et 60 $cm^3$ d'éthanol. L'agitation est encore maintenue 4 heures à température ambiante puis le mélange réactionnel est abandonné une nuit. Le lendemain l'éthanol est éliminé par évaporation sous vide et la suspension obtenue est reprise par 200 $cm^3$ d'eau puis extraite trois fois par 200 $cm^3$ d'acétate d'éthyle. Les phases d'acétate d'éthyle rassemblées sont lavées par une solution aqueuse saturée de chlorure d'ammonium et séchées sur sulfate de magnésium. L'acétate d'éthyle est éliminé par évaporation sous vide et les 16 g de produit

jaune obtenus sont purifiés par chromatographie sur gel de silice. Le produit attendu est élué au mélange hexane-acétate d'éthyle (9,5-0,5). Après évaporation de l'éluant et recristallisation dans l'hexane contenant des traces de toluène, on obtient 7,2 g de méthyl [E(tétrahydro-5,6,7,8 tétraméthyl-5,5,8,8 naphtyl-2)-3 oxo-3 propényl]-4 phénylthioéther sous forme d'une poudre jaune de point de fusion : 124°C.
Analyse élémentaire : $C_{24}H_{28}OS$

| | C | H | S |
|---|---|---|---|
| Calculé | 79,07 | 7,74 | 8,80 |
| Trouvé | 78,95 | 7,74 | 8,53 |

EXEMPLE VII

Préparation du méthyl[E(tétrahydro-5,6,7,8 tétraméthyl-5,5,8,8 naphtyl-2)-3 oxo-3 propényl]-4 phényl sulfoxyde

A une solution agitée à 0°C à l'abri de la lumière de 2 g de méthyl[E(tétrahydro-5,6,7,8 tétraméthyl-5,5,8,8 naphtyl-2)-3 oxo-3 propényl]-4 phénylthioéther obtenu à l'exemple VI dans 100 cm³ de dichlorométhane on ajoute par petites portions un équivalent d'acide métachloroperbenzoïque. La transformation du thioéther en sulfoxyde est suivie en chromatographie couche mince (C.C.M.). Lorsqu'elle est totale, le mélange réactionnel est versé sur une solution aqueuse saturée de chlorure d'ammonium puis extrait trois fois par 100 cm³ de dichlorométhane. Les phases organiques sont rassemblées, lavées par une solution aqueuse de bicarbonate de sodium, séchées sur sulfate de magnésium, concentrées puis déposées sur une colonne de chromatographie de gel de silice. Le sulfoxyde attendu est élué à l'acétate d'éthyle.

Après concentration de l'éluant et recristallisation dans l'hexane, on obtient 1,4 g de méthyl [E(tétrahydro-5,6,7,8 tétraméthyl-5,5,8,8 naphtyl-2)-3 oxo-3 propényl]-4 phénylsulfoxyde sous forme de cristaux blancs de point de fusion : 116°C.
Analyse élémentaire : $C_{24}H_{28}O_2S$

| | C | H | O | S |
|---|---|---|---|---|
| Calculé | 75,75 | 7,41 | 8,41 | 8,43 |
| Trouvé | 75,45 | 7,44 | 8,88 | 8,02 |

EXEMPLE VIII

Préparation du N-éthyl[E(tétrahydro-5,6,7,8 tétraméthyl-5,5,8,8 naphtyl-2)-3 oxo-3 propényl]-4 phényl sulfonamide

A une solution agitée à température ordinaire à l'abri de la lumière de 0,6 g de potasse dans 20 cm³ d'eau et 10 cm³ d'éthanol, on ajoute rapidement un mélange de 1,05 g d'acétyl-2 tétrahydro-5,6,7,8 tétraméthyl-5,5,8,8 naphtalène et de 0,95 g de N-éthyl formyl-4 phényl sulfonamide dissous dans 20 cm³ d'éthanol. L'addition terminée, l'agitation est encore maintenue 4 heures et le mélange réactionnel est abandonné une nuit. L'éthanol est évaporé sous pression réduite et le produit obtenu est agité dans 200 cm³ d'eau puis extrait trois fois par 150 cm³ d'acétate d'éthyle. Les phases organiques rassemblées sont lavées par une solution aqueuse saturée de chlorure d'ammonium et séchées sur sulfate de magnésium. L'acétate d'éthyle est éliminé par évaporation sous vide et on obtient 1,3 g de solide que l'on recristallise dans un mélange toluène-hexane (1-1).

Le N-éthyl[E(tétrahydro-5,6,7,8 tétraméthyl-5,5,8,8 naphtyl-2)-3 oxo-3 propényl]-4 phényl sulfonamide est un solide blanc de point de fusion : 147°C.
Analyse élémentaire : $C_{25}H_{31}NO_3S$

| | C | H | N | O | S |
|---|---|---|---|---|---|
| Calculé | 70,55 | 7,34 | 3,29 | 11,28 | 7,53 |
| Trouvé | 70,59 | 7,35 | 3,25 | 11,06 | 7,48 |

EXEMPLE IX

Préparation du méthyl[E(pentaméthyl-1,1,2,3,3 indanyl-5)-3 oxo-3 propényl]-4 phénylthioéther

De la même façon qu'à l'exemple VI, un mélange de 10 g d'acétyl-5 pentaméthyl-1,1,2,3,3 indane et de 6,5 g de méthylthio-4 benzaldéhyde solubilisé dans 140 cm³ d'éthanol sont ajoutés à l'abri de la lumière à une solution de 5,8 g de potasse dans un mélange de 160 cm³ d'eau et 60 cm³ d'éthanol. A la fin de la réaction, le mélange réactionnel est traité suivant l'exemple VI.

Le thiother attendu est purifié par passage sur colonne de gel de silice en utilisant comme éluant un mélange hexane acétate d'éthyle (9-1). Après évaporation de l'éluant, on recristallise dans un mélange hexane-toluène et on obtient 5 g de méthyl[E(pentaméthyl-1,1,2,3,3 indanyl-5)-3 oxo-3 propényl]-4 phénylthioéther sous forme de cristaux jaune clair de point de fusion : 109°C.
Analyse élémentaire : $C_{24}H_{28}OS$

| | C | H | S |
|---|---|---|---|
| Calculé | 79,07 | 7,74 | 8,80 |
| Trouvé | 78,72 | 7,74 | 8,52 |

EXEMPLE X

Préparation du méthyl[E(pentaméthyl-1,1,2,3,3 indanyl-5)-3 oxo-3 propényl]-4 phényl sulfoxyde

A une solution agitée à 0°C à l'abri de la lumière de 2,3 g de méthyl [E(pentaméthyl-1,1,2,3,3 indanyl-5)-3 oxo-3 propényl]-4 phénylthioéther, préparé selon l'exemple IX dans 100 cm³ de dichlorométhane, on ajoute par petite portion un équivalent d'acide métachloroperbenzoïque. Après la fin de l'addition, le milieu réactionnel est encore agité deux heures à 0°C puis une heure à température ambiante temps au bout duquel la totalité du thioéther est transformée. Le mélange est alors versé sur 200 cm³ d'une solution aqueuse de chlorure d'ammonium et extrait trois fois par 100 cm³ de dichlorométhane et les phases organiques rassemblées sont lavées avec une solution aqueuse de bicarbonate de sodium puis séchées sur sulfate de magnésium et enfin concentrées.

Le produit brut est purifié par chromatographie sur gel de silice en utilisant comme éluant de l'acétate d'éthyle. Après évaporation de l'éluant, le résidu est recristallisé dans le cyclohexane et on obtient 0,9 g de méthyl[E(pentaméthyl-1,1,2,3,3 indanyl-5)-3 oxo-3 propényl]-4 phényl sulfoxyde sous la forme de cristaux blancs de point de fusion : 113°C.
Analyse élémentaire : $C_{24}H_{28}O_2S$

| | C | H | O | S |
|---|---|---|---|---|
| Calculé | 75,75 | 7,41 | 8,41 | 8,43 |
| Trouvé | 75,64 | 7,40 | 8,50 | 8,26 |

EXEMPLE XI

Préparation de la méthyl[E(pentaméthyl-1,1,2,3,3 indanyl-5)-3 oxo-3 propényl]-4 phényl sulfone

A une solution de 2,3 g de potasse dans un mélange de 30 cm³ d'éthanol et 80 cm³ d'eau agitée à l'abri de la lumière à température ambiante, on ajoute rapidement un mélange de 4,6 g d'acétyl-2 pentaméthyl-1,1,2,3,3 indane et de 3,7 g de méthylsulfonyl-4 benzaldéhyde dans 70 cm³ d'éthanol. Après 4 heures d'agitation à température ambiante, la réaction est terminée.

L'éthanol est évaporé sous pression réduite et la suspension obtenue est reprise par 200 cm³ d'eau puis extraite trois fois par 150 cm³ d'acétate d'éthyle. Les phases d'acétate d'éthyle sont rassemblées, lavées avec une solution aqueuse saturée de chlorure d'ammonium, séchées sur sulfate de magnésium et concentrées. Le solide obtenu est recristallisé deux fois dans l'hexane en présence d'une trace de toluène. On obtient 2,1 g de méthyl E(pentaméthyl-1,1,2,3,3 indanyl-5)-3 oxo-3 propényl -4 phényl sulfone sous forme de cristaux blancs de point de fusion : 119°C.

Analyse élémentaire : $C_{24}H_{28}O_3S$

| | C | H | O | S |
|---|---|---|---|---|
| Calculé | 72,69 | 7,12 | 12,10 | 8,09 |
| Trouvé | 72,28 | 7,13 | 12,34 | 7,88 |

## EXEMPLE XII

### Préparation de la méthyl[E(pentaméthyl-1,1,2,3,3 indanyl-5)-3 hydroxy-3 propényl]-4 phényl sulfone

A une suspension de 1 g de méthyl[E(pentaméthyl-1,1,2,3,3 indanyl-5)-3 oxo-3 propényl]-4 phényl sulfone obtenu à l'exemple XI dans 80 cm³ de méthanol agitée à l'abri de lumière à 0°C on ajoute en une fois 0,1 g de borohydrure de sodium.

L'agitation est maintenue 4 heures à cette température puis le milieu réactionnel est ensuite abandonné pendant 48 heures à température ambiante. Le méthanol est ensuite éliminé par évaporation sous vide et le produit brut est repris par 200 cm³ d'eau. On extrait trois fois par 100 cm³ d'acétate d'éthyle et les phases d'acétate d'éthyle sont rassemblées, lavées avec une solution aqueuse saturée de chlorure d'ammonium, séchées sur sulfate de magnésium et concentrées sous pression réduite. Le produit brut est déposé sur une colonne de chromatographie de gel de silice, en utilisant comme éluant l'acétate d'éthyle. Après concentration, on obtient 0,5 g de méthyl[E(pentaméthyl-1,1,2,3,3 indanyl-5)-3 hydroxy-3 propényl]-4 phényl sulfone sous forme d'une poudre blanche de point de fusion : 68°C.

Analyse élémentaire : $C_{24}H_{30}O_3S$

| | C | H | O | S |
|---|---|---|---|---|
| Calculé | 72,32 | 7,59 | 12,04 | 8,05 |
| Trouvé | 72,17 | 7,79 | 11,97 | 7,75 |

## EXEMPLE XIII

### Préparation de la méthyl[E(tétrahydro-5,6,7,8 tétraméthyl-5,5,8,8 naphtyl-2)-3 méthoxy-3 propényl]-4 phényl sulfone

A une suspension de 1 g de méthyl[E(tétrahydro-5,6,7,8 tétraméthyl-5,5,8,8 naphtyl-2)-3 oxo-3 propényl]-4 phényl sulfone obtenu selon l'exemple V dans 80 cm³ de méthanol agitée à l'abri de la lumière à 0°C, on ajoute en une fois 0,1 g de borohydrure de sodium. L'agitation est maintenue 4 heures à cette température puis le méthanol est ensuite éliminé par évaporation sous pression réduite. Le produit brut obtenu est repris par 200 cm³ d'eau et la suspension extraite trois fois par 100 cm³ d'acétate d'éthyle. Les phases d'acétate d'éthyle sont rassemblées, lavées avec une solution aqueuse saturée de chlorure d'ammonium, séchées sur sulfate de magnésium et concentrées sous pression réduite.

On récupère 0,5 g de méthyl [E(tétrahydro-5,6,7,8 tétraméthyl-5,5,8,8 naphtyl-2)-3 hydroxy-3 propényl]-4 phényl sulfone brut qui sont remis en solution dans 30 cm³ de méthanol en présence d'acide paratoluène sulfonique comme catalyseur. La solution est portée sous reflux pendant 2 heures à l'abri de la lumière. Le méthanol est évaporé sous pression réduite et le produit brut est purifié par passage sur colonne de chromatographie de gel de silice. L'éther méthylique attendu est élué au mélange, heptane-acétate d'éthyle (8-2) puis, après évaporation de l'éluant, recristallisé dans l'hexane.

On obtient 0,3 g de méthyl [E(tétrahydro-5,6,7,8 tétraméthyl-5,5,8,8 naphtyl-2)-3 méthoxy-3 propényl]-4 phényl sulfone sous forme de cristaux blancs de point de fusion : 104°C.

Analyse élémentaire : $C_{25}H_{32}O_3S$

| | C | H | O | S |
|---|---|---|---|---|
| Calculé | 72,77 | 7,82 | 11,63 | 7,77 |
| Trouvé | 72,38 | 8,09 | 12,02 | 7,50 |

EXEMPLE XIV

Préparation de l'éthyl[(tétrahydro-5,6,7,8 tétraméthyl-5,5,8,8 naphtyl-2)carbonyl]-6 naphtyl sulfone-2

A une solution agitée à l'abri de l'humidité de l'air de 1 g de chlorure d'acide éthylsulfonyl-6 naphtalène carboxylique-2 préparé selon l'exemple D dans 70 $cm^3$ de dichloro-1,2 éthane anhydre, on ajoute 0,75 g de tétrahydro-1,2,3,4 tétraméthyl-1,1,4,4 naphtalène, puis à 0°C par petites portions 0,75 g de chlorure d'aluminium.

A la fin de l'addition, on maintient l'agitation encore une heure à température ambiante. Le milieu réactionnel est alors versé dans 100 $cm^3$ d'une solution aqueuse saturée de chlorure d'ammonium, puis extraite trois fois par 100 $cm^3$ de chlorure de méthylène. Les phases organiques rassemblées sont lavées au bicarbonate de sodium, puis à l'eau, séchées sur sulfate de magnésium et concentrées.

Le produit brut est recristallisé dans un mélange hexane-éther isopropylique. On obtient 0,9 g d'éthyl [(tétrahydro-5,6,7,8 tétraméthyl-5,5,8,8 naphtyl-2)carbonyl]-6 naphtyl sulfone -2 sous forme de cristaux blancs de point de fusion : 145°C.
Analyse élémentaire : $C_{27}H_{30}O_3S$

| | C | H | O | S |
|---|---|---|---|---|
| Calculé | 74,62 | 6,96 | 11,04 | 7,38 |
| Trouvé | 73,95 | 7,00 | 11,78 | 7,06 |

EXEMPLE XV

Préparation de l'éthyl[(pentaméthyl-1,1,2,3,3 indanyl-5)carbonyl]-6 naphtyl sulfone-2

A un mélange agité à 0°C à l'abri de l'humidité de l'air de 1,1 g de pentaméthyl-1,1,2,3,3 indane et de 1,5 g de chlorure d'acide éthylsulfonyl-6 naphtalène carboxylique-2 obtenu selon l'exemple D dans 70 $cm^3$ de dichloro-1,2 éthane anhydre, on ajoute par petites portions 1,1 g de chlorure d'aluminium. Après la fin de l'addition, le mélange est agité une heure à 0°C puis une heure à température ambiante. On verse alors sur 100 $cm^3$ d'une solution saturée de chlorure d'ammonium, et on extrait 3 fois avec 100 $cm^3$ de dichlorométhane. Les phases organiques sont rassemblées, lavées au bicarbonate de sodium puis à l'eau et enfin séchées sur sulfate de magnésium. Après évaporation du solvant, on obtient 1,5 g d'un produit brut qui est recristallisé dans un mélange hexane-toluène (1-1) avec une trace d'éthanol.

L'éthyl [(pentaméthyl-1,1,2,3,3 indanyl-5)carbonyl]-6 naphtyl sulfone –2 est une poudre blanche de point de fusion : 136°C.
Analyse élémentaire : $C_{27}H_{30}O_3S$

| | C | H | O | S |
|---|---|---|---|---|
| Calculé | 74,62 | 6,96 | 11,04 | 7,38 |
| Trouvé | 74,42 | 6,95 | 11,06 | 7,45 |

EXEMPLE XVI

Préparation de l'éthyl[(tétrahydro-5,6,7,8 tétraméthyl-5,5,8,8 naphtyl-2)-hydroxyméthyl]-6 naphtylsulfone-2

A une suspension de 200 mg d'éthyl [(tétrahydro-5,6,7,8 tétraméthyl-5,5,8,8 naphtyl-2)carbonyl]-6 naphtylsulfone-2, obtenu selon l'exemple XIV, dans 30 $cm^3$ de méthanol refroidie à 0°C, on ajoute 100 mg

de borohydrure de sodium. On maintient le milieu réactionnel sous agitation pendant 30 minutes puis on laisse revenir à température ambiante. Après vérification en chromatographie sur couche mince de la disparition du produit carbonylé de départ, l'alcool est évaporé sous pression réduite et le résidu est repris dans 100 $cm^3$ d'eau et acidifié avec une solution d'acide chlorhydrique 1N. Le précipité obtenu est filtré et séché sous vide.

Après recristallisation dans un mélange toluène-hexane, on récupère 150 mg de cristaux blancs fondant à 157°C et dont le spectre $^1H$ RMN 80MHz est conforme à la structure attendue.
Analyse élémentaire : $C_{27}H_{32}O_3S$

| | C | H | O | S |
|---|---|---|---|---|
| Calculé | 74,27 | 7,39 | 11,00 | 7,34 |
| Trouvé | 74,10 | 7,35 | 11,19 | 7,44 |

EXEMPLE XVII

Préparation de l'éthyl[(tétrahydro-5,6,7,8 tétraméthyl-5,5,8,8 naphtyl-2)-1 hydroxy-1 éthyl]-6 naphtylthioéther-2

A une solution de 6,9 g de bromo-2 tétrahydro-5,6,7,8 tétraméthyl-5,5,8,8 naphtalène dans 50 $cm^3$ de tétrahydrofuranne anhydre, on ajoute 0,75 g de magnésium. La préparation de l'organomagnésien est démarrée par chauffage local puis la solution est maintenue au reflux du T.H.F. jusqu'à consommation totale du magnésium.

Le milieu réactionnel est alors refroidi à 0°C, puis on ajoute, goutte à goutte, 3 g d'acétyl-2 éthylthio-6 naphtalène, obtenu selon l'exemple C (2), en solution dans 50 $cm^3$ de T.H.F.. A la fin de l'addition, le mélange réactionnel est maintenu à 0°C pendant 2 heures puis à température ambiante pendant une nuit. Après vérification en chromatographie sur couche mince de la disparition du produit de départ, le milieu réactionnel est versé sur 200 $cm^3$ d'eau et extrait à l'éther éthylique. La phase organique est lavée avec 100 $cm^3$ d'une solution d'hydrogènocarbonate de sodium, puis à l'eau. Après concentration sous pression réduite, on récupère 3,6 g d'une huile jaune qui cristallise dans l'hexane. Après recristallisation dans l'hexane, on récupère 2 g du produit attendu de point de fusion : 122°C.
Analyse élémentaire : $C_{28}H_{34}OS$

| | C | H | O | S |
|---|---|---|---|---|
| Calculé | 80,33 | 8,19 | 3,82 | 7,66 |
| Trouvé | 80,33 | 8,26 | 4,60 | 7,67 |

EXEMPLE XVIII

Préparation de l'éthyl[(tétrahydro-5,6,7,8 tétraméthyl-5,5,8,8 naphtyl-2)-1 éthényl]-6 naphtylthioéther-2

A une solution de 1,6 g d'éthyl[(tétrahydro-5,6,7,8 tétraméthyl-5,5,8,8 naphtyl-2)-1 hydroxy-1 éthyl]-6 naphtylthioéther-2, obtenu selon l'exemple XVII, dans 100 $cm^3$ de toluène, on ajoute 1 g d'acide para-toluène sulfonique. Le mélange réactionnel est maintenu sous agitation pendant 1 heure à 70°C et abandonné pendant une nuit à température ambiante. Après hydrolyse avec 200 $cm^3$ d'eau, le produit attendu est extrait à l'acétate d'éthyle. La phase organique est lavée avec une solution de bicarbonate de sodium, séchée sur sulfate de magnésium puis concentrée sous pression réduite. Après purification par chromatographie sur gel de silice (éluant : heptane-acétate d'éthyle 9,5 - 0,5) on récupère 500 mg d'une huile jaune qui cristallise dans l'hexane et dont le point de fusion est de 102°C.
Analyse élémentaire : $C_{28}H_{32}S$

| | C | H | S |
|---|---|---|---|
| Calculé | 83,94 | 8,05 | 8,01 |
| Trouvé | 84,09 | 8,11 | 7,85 |

## EXEMPLE XIX

### Préparation de l'éthyl[(diméthyl-5,8 méthoxy-6 naphtyl-2)carbonyl]-6 naphtylsulfone-2

Une suspension de 3 g (11,35 mmoles) d'acide éthylsulfonyl-6 naphtalène carboxylique, décrit à l'exemple D(1), dans 8,5 cm³ (10 éq.) de chlorure de thionyle est chauffée 1 heure au reflux. La solution obtenue est évaporée à sec sous pression réduite. Le chlorure d'acide brut ainsi isolé (solide beige) est repris par 30 cm³ de dichloro-1,2 éthane sec. On ajoute 2,12 g (11,38 mmoles) de diméthyl-5,8 méthoxy-6 naphtalène puis par portions 2,27 g (17 mmoles) de chlorure d'aluminium anhydre et agite 3 heures à température ambiante. Le milieu réactionnel est alors versé sur 30 cm³ d'eau glacée. On dilue par 50 cm³ de dichloroéthane, décante la phase organique et réextrait la phase aqueuse par 30 cm³ de dichloroéthane. Les phases organiques sont réunies, lavées à l'eau, séchées sur sulfate de sodium et évaporées à sec. Le solide brut isolé est purifié par chromatographie sur gel de silice 60 dans le dichlorométhane suivie d'une recristallisation dans un mélange toluène/hexane. Après séchage, on obtient 1,5 g de cristaux jaune pâle de l'éthyl[(diméthyl-5,8 méthoxy-6 naphtyl-2)carbonyl]-6 naphtylsulfone-2 de point de fusion : 164-165°C.

Le spectre RMN¹H 250MHz est conforme à la structure attendue.

Analyse élémentaire : $C_{26}H_{24}O_4S$

| | C | H | O | S |
|---|---|---|---|---|
| Calculé | 72,20 | 5,59 | 14,80 | 7,41 |
| Trouvé | 72,36 | 5,58 | 14,36 | 7,03 |

## EXEMPLE XX

### Préparation de l'éthyl[(diméthyl-5,8 méthoxy-6 naphtyl-2) hydroxyméthyl]-6 naphtylsulfone-2

A une solution de 0,5 g (1,16 mmoles) de l'éthyl[(diméthyl-5,8 méthoxy-6 naphtyl-2)carbonyl]-6 naphtylsulfone-2, décrit à l'exemple XIX, on ajoute 0,175 g (4,64 mmoles) de borohydrure de sodium et agite 3 heures à température ambiante. On refroidit alors à 0°C puis acidifie par addition lente d'acide chlorhydrique 0,1N et extrait à l'éther éthylique (3 × 40 cm³). La phase éthérée est lavée à l'eau, séchée sur sulfate de sodium et évaporée à sec. Le solide brut isolé est purifié par chromatographie sur gel de silice 60 dans le dichlorométhane suivie d'une recristallisation dans l'hexane contenant une trace d'acétone. On obtient après séchage 0,3 g de l'éthyl[(diméthyl-5,8 méthoxy-6 naphtyl-2)hydroxyméthyl]-6 naphtylsulfone-2 sous la forme de cristaux beige de point de fusion : 141-143°C.

Le spectre RMN¹H 250MHz est conforme à la structure attendue.

Analyse élémentaire : $C_{26}H_{26}O_4S$

| | C | H | O | S |
|---|---|---|---|---|
| Calculé | 71,86 | 6,03 | 14,73 | 7,38 |
| Trouvé | 71,55 | 6,34 | 14,44 | 6,99 |

## EXEMPLE XXI

### Préparation de l'éthyl[(diméthyl-5,8 méthoxy-6 naphtyl-2) méthylène]-6 naphtylsulfone-2

Une suspension de 0,43 g (1 mmole) de l'éthyl[(diméthyl-5,8 méthoxy-6 naphtyl-2)carbonyl]-6 naphtylsulfone-2, décrit à l'exemple XIX, dans 10 cm³ d'acide acétique glacial et 0,65 g de zinc en poudre (10 mmoles), est chauffée au reflux sous agitation. On ajoute ensuite goutte à goutte 0,85 cm³ (10 mmoles) d'acide

chlorhydrique 12N et maintient le reflux 1 heure. On ajoute à nouveau 0,85 cm³ d'acide chlorhydrique 12N et agite 1 heure en laissant refroidir. Après addition de 20 cm³ d'acide chlorhydrique 12N, on extrait à l'éther (3 × 30 cm³), lave à l'eau, sèche sur sulfate de sodium et évapore à sec. Le solide jaune brut est purifié rapidement par chromatographie sur gel de silice 60 (éluant : dichlorométhane) suivie d'une recristallisation dans un mélange hexane/acétone. Après séchage, on obtient 160 mg d'aiguilles blanches de l'éthyl (diméthyl-5,8 méthoxy-6 naphtyl-2) méthylène -6 naphtylsulfone-2 de point de fusion : 166°C.

Le spectre RMN¹H 80MHz est conforme à la structure attendue.

EXEMPLE XXII

Préparation de l'éthyl[(tétrahydro-5,6,7,8 tétraméthyl-5,5,8,8 naphtyl-2)méthylène]-6 naphtylsulfone-2.

Une suspension de 0,50 g d'éthyl[(tétrahydro-5,6,7,8 tétraméthyl-5,5,8,8 naphtyl-2) carbonyl]-6 naphtylsulfone-2, décrit à l'exemple XIV, dans 10 cm³ d'acide acétique glacial et 0,65 g de zinc en poudre est chauffée au reflux sous agitation pendant une heure.

Puis on ajoute, goutte à goutte, 0,9 cm³ d'acide chlorhydrique concentré. Le reflux est encore maintenu pendant une heure après la fin de l'addition de l'acide chlorhydrique et on ajoute de nouveau 0,9 cm³ d'acide chlorhydrique et le mélange est porté pendant encore une heure à la température d'ébullition de l'acide acétique puis abandonné pendant la nuit. Le lendemain on verse dans 20 cm³ d'acide chlorhydrique 6 N et extrait trois fois par 30 cm³ d'éther éthylique. Les phases éthérées rassemblées sont lavées à l'eau, décantées et séchées sur sulfate de sodium. Après évaporation du solvant on obtient 0,5 g de poudre jaune que l'on dissout dans du chlorure de méthylène et la solution est déposée sur une colonne de chromatographie de gel de silice.

Après concentration des fractions contenant le produit attendu pur et séchage on obtient 0,32 g d'éthyl[(tétrahydro-5,6,7,8 tétraméthyl-5,5,8,8 naphtyl-2) méthylène]-6 naphtylsulfone-2 sous forme de cristaux blancs dont le point de fusion est de 174°C.

Le spectre de ¹H R.M.N. correspond à la structure attendue.

EXEMPLES DE COMPOSITIONS

A - VOIE ORALE

Exemple 1 - Comprimé de 0,2g

- Ethyl[(tétrahydro-5,6,7,8 tétraméthyl-5,5,8,8 naphtyl-2) carbonyl]-6 naphtylsulfone-2 .............................. 0,010g
- Amidon .............................................. 0,115g
- Phosphate bicalcique .............................. 0,020g
- Silice .............................................. 0,020g
- Lactose .............................................. 0,030g
- Talc .............................................. 0,010g
- Stéarate de magnésien .............................. 0,005g

Exemple 2 - Suspension buvable en ampoule 5ml

- Ethyl (pentaméthyl-1,1,2,3,3 indanyl-5)carbonyl -6 naphtylsulfone-2..........0,010g
- Glycérine..........0,500g
- Sorbitol à 70%..........0,500g
- Saccharinate de sodium..........0,010g
- Parahydroxybenzoate de méthyle..........0,040g
- Arôme.......... q.s.
- Eau purifiée q.s.p..........5,000g

## B - VOIE TOPIQUE

Exemple 3 - Onguent

- Ethyl[(tétrahydro-5,6,7,8 tétraméthyl-5,5,8,8 naphtyl-2) carbonyl]-6 naphtylsulfone-2..........0,010g
- Huile de vaseline fluide..........9,100g
- Silice vendue par la Société DEGUSSA sous la dénomination de "Aérosil 200"..........9,100g
- Myristate d'isopropyle q.s.p..........100,000g

Exemple 4 - Crème huile-dans-l'eau anionique

- Ethyl[(pentaméthyl-1,1,2,3,3 indanyl-5)carbonyl]-6 naphtylsulfone-2..........0,100g
- Dodécyl sulfate de sodium..........0,800g
- Glycérol..........2,000g
- Alcool stéarylique..........20,000g
- Triglycérides d'acides caprique/caprylique vendus par la Société DYNAMIT NOBEL sous la dénomination de "Miglyol 812"..........20,000g
- Conservateurs.......... q.s.
- Eau déminéralisée q.s.p..........100,000g

Exemple 5 - Gel

- Ethyl [(pentaméthyl-1,1,2,3,3 indanyl-5)carbonyl]-6 naphtylsulfone-2..........0,500g
- Hydroxypropyl cellulose vendue par la Société HERCULES sous le nom de "KLUCEL HF"..........2,000g
- Eau/éthanol (50/50) q.s.p..........100,000g

Exemple 6 - Crème anti-séborrhéique

- Stéarate de polyoxyéthylène (40 moles d'oxyde d'éthylène) vendu sous la dénomination de "MYRJ 52" par la Société ATLAS....................4,000g
- Mélange d'esters laurique de sorbitol et de sorbitan polyoxyéthyléné à 20moles d'oxyde d'éthylène vendu sous la dénomination de "TWEEN 20" par la Société "ATLAS"....................1,800g
- Mélange de mono- et distéarate de glycérol vendu sous la dénomination de "GELEOL" par la Société GATTEFOSSE....................4,200g
- Propylèneglycol....................10,000g
- Butylhydroxyanisole....................0,010g
- Butylhydroxytoluène....................0,020g
- Alcool céto-stéarylique....................6,200g
- Conservateurs.................... q.s.
- Perhydrosqualène....................18,000g
- Mélange de triglycérides caprylique/caprique vendu sous la dénomination de "MIGLYOL 812" par la Société DYNAMIT NOBEL....................4,000g
- S-carboxyméthyl cystéine....................3,000g
- Triéthanolamine 99%....................2,500g
- Méthyl[E(pentaméthyl-1,1,2,3,3 indanyl-5)-3 hydroxy-3 propényl]-4 phénylsulfone....................0,100g
- Eau q.s.p....................100,000g

Exemple 7 - Crème anti-séborrhéique

- Stéarate de polyoxyéthylène (40 moles d'oxyde d'éthylène) vendu sous la dénomination de "MYRJ 52" par la Société ATLAS....................4,000g
- Mélange d'esters laurique de sorbitol et de sorbitan polyoxyéthyléné à 20 moles d'oxyde d'éthylène vendu sous la dénomination de "TWEEN 20" par la Société ATLAS....................1,800g
- Mélange de mono- et distéarate de glycérol vendu sous la dénomination de "GELEOL" par la Société GATTEFOSSE....................4,200g
- Propylèneglycol....................10,000g

Dans cet exemple, le composé actif peut être remplacé par 0,05 g d'éthyl [(tétrahydro-5,6,7,8 tétraméthyl-5,5,8,8 naphtyl-2)hydroxydrométhyl]-6 naphtylsulfone-2.

- Butylhydroxyanisole..........................................0,010g
- Butylhydroxytoluène..........................................0,020g
- Alcool cétostéarylique......................................6,200g
- Conservateurs.................................................. q.s.p.
- Perhydrosqualène............................................18,000g
- Mélange de triglycérides caprylique/caprique vendu sous la dénomination de "MIGLYOL 812" par la Société DYNAMIT NOBEL..............................4,000g
- Amino-5 carboxy-5 thia-3 pentanoate de benzylthio-2 éthylammonium................................3,000g
- Ethyl [(tétrahydro-5,6,7,8 tétraméthyl-5,5,8,8 naphtyl-2)carbonyl]-4 naphtylsulfone-2.....................0,500g
- Eau q.s.p...................................................100,000g

Exemple 8 - Lotion pour les cheveux

- Propylèneglycol.............................................20,000g
- Ethanol.........................................................34,870g
- Polyéthylèneglycol de masse moléculaire 400..............40,000g
- Eau................................................................4,000g
- Butylhydroxyanisole..........................................0,010g
- Butylhydroxytoluène..........................................0,020g
- Ethyl [(tétrahydro-5,6,7,8 tétraméthyl-5,5,8,8 naphtyl-2)carbonyl]-6 naphtylsulfone-2....................0,100g
- Minoxidil.........................................................1,000g

Exemple 9 - Gel anti-acné

- Ethyl [(tétrahydro-5,6,7,8 tétraméthyl-5,5,8,8 naphtyl-2)carbonyl]-6 naphtylsulfoxyde-2.................0,100g
- Alcool isopropylique....................................40,000g
- Polymère de l'acide acrylique vendu sous la dénomination de "CARBOPOL 940" par la Société GOODRICH CHEMICAL COMPANY........................1,000g
- Triéthanolamine 99%.....................................0,600g
- Butylhydroxyanisole.....................................0,010g
- Butylhydroxytoluène.....................................0,020g
- Tioxolone...............................................0,500g
- Propylèneglycol.........................................8,000g
- Eau purifiée q.s.p....................................100,000g

## Revendications

**Pour les états contractants : AT, BE, CH, DE, FR, GB, IT, LI, NL, SE**

1. Composés polycycliques aromatiques caractérisés par le fait qu'ils répondent à la formule suivante:

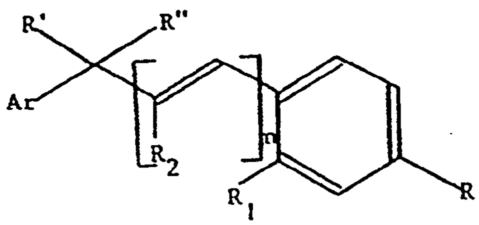

(I)

dans laquelle :

R représente

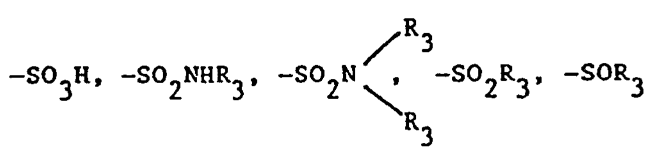

ou $-SR_3$,

$R_3$ représentant un radical alkyle inférieur, linéaire ou ramifié, un radical monohydroxyalkyle, polyhydroxyalkyle, aminoalkyle, carboxyalkyle, aminocarboxyalkyle ou alkoxyalkyle,

R' représente un atome d'hydrogène, un radical alkyle inférieur, un radical alkoxy inférieur, un radical hydroxyle, un radical acyloxy en $C_1$-$C_4$ ou un radical amino,

R" représente un atome d'hydrogène, un radical alkyle inférieur ou un radical alkoxy inférieur, ou

R' et R", pris ensemble, forment un radical oxo (=O), méthano (=$CH_2$) ou hydroxyimino (=N–OH),

$R_1$ représente un atome d'hydrogène ou un radical alkyle inférieur,

n est 0 ou 1,

lorsque n=1, $R_2$ représente un atome d'hydrogène ou un radical alkyle inférieur, ou $R_1$ et $R_2$, pris ensemble,

forment un radical vinylène (-CH=CH-), et

Ar représente un radical aromatique selon l'une des formules suivantes :

(i)

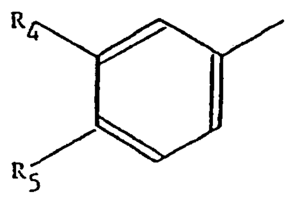

(ii)

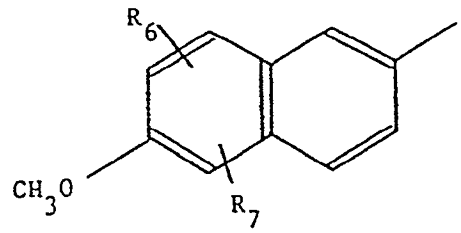

(iii)

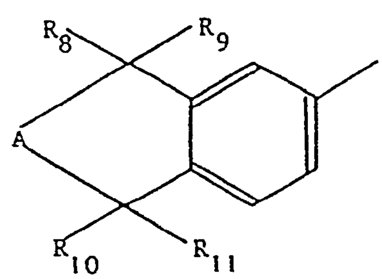

dans lesquelles :

$R_4$, $R_5$, $R_6$, $R_7$, $R_8$, $R_9$, $R_{10}$ et $R_{11}$ représentent un atome d'hydrogène ou un radical alkyle inférieur, au moins un des radicaux $R_4$ et/ou $R_5$ étant différent d'un atome d'hydrogène, et au moins deux des radicaux $R_8$ à $R_{11}$ étant différents d'un atome d'hydrogène,

A représente un radical méthylène ou diméthylène substitué ou non par un radical alkyle inférieur ; lorsque A représente un radical diméthylène, $R_8$ et $R_{10}$ peuvent former ensemble un radical méthylène ou diméthylène,

et les sels desdits composés de formule (I) ainsi que leurs isomères géométriques et optiques.

2. Composés selon la revendication 1 caractérisés par le fait que le radical alkyle inférieur est pris dans le groupe constitué par le radical méthyle, éthyle, isopropyle, butyle et tertiobutyle.

3. Composés selon la revendication 1 caractérisés par le fait que le radical monohydroxyalkyle est un radical ayant de 2 à 6 atomes de carbone, notamment le radical hydroxy-2 éthyle ou le radical hydroxy-2 propyle.

4. Composés selon la revendication 1 caractérisés par le fait que le radical polyhydroxyalkyle est un radical ayant de 3 à 6 atomes de carbone et de 2 à 5 groupes hydroxyles, notamment le radical dihydroxy-2,3 propyle, dihydroxy-1,3 propyl-2 ou le reste du pentaérythritol.

5. Composés selon la revendication 1 caractérisés par le fait que le radical aminoalkyle comporte de 2 à 6 atomes de carbone tels que les radicaux amino-2 éthyle, amino-2 propyle ou amino-3 propyle.

6. Composés selon la revendication 1 caractérisés par le fait que le radical carboxyalkyle comporte de 2 à 7 atomes de carbone tels que les radicaux carboxyméthyle, carboxy-2 éthyle, carboxy-2 propyle ou carboxy-3 butyle.

7. Composés selon la revendication 1 caractérisés par le fait que le radical aminocarboxyalkyle est le radical amino-2 carboxy-2 éthyle ou amino-3 carboxy-3 propyle.

8. Composés selon la revendication 1 caractérisés par le fait que le radical alkoxy inférieur est le radical méthoxy, éthoxy, butoxy ou isopropoxy.

9. Composés selon l'une quelconque des revendications 1 à 8 caractérisés par le fait qu'ils répondent

à la formule suivante :

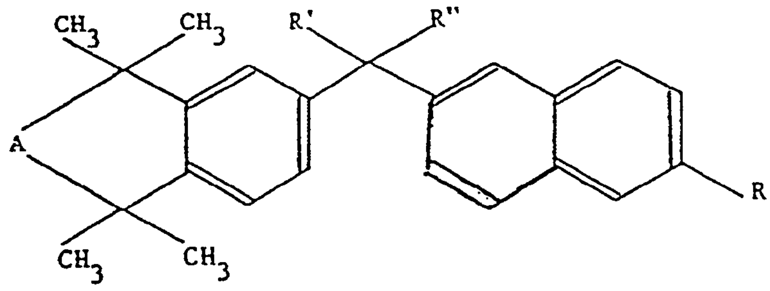

(II)

dans laquelle :

R représente $-SO_2R'_3$, $SO_2NHR'_3$, $SOR'_3$ ou $-SR'_3$,

$R'_3$ représentant un radical alkyle inférieur,

R′ représente un atome d'hydrogène, un radical hydroxyle ou alkyle inférieur,

R″ représente un atome d'hydrogène ou R′ et R″ pris ensemble forment un radical oxo (=O), et

A représente un radical diméthylène ou un radical de formule

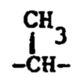

10. Composés selon l'une quelconque des revendications 1 à 9 caractérisés par le fait qu'ils sont pris dans le groupe constitué par :

- La méthyl[(tétrahydro-5,6,7,8 tétraméthyl-5,5,8,8 naphtyl-2) hydroxyméthyl]-4 phénylsulfone,
- La méthyl[(tétrahydro-5,6,7,8 tétraméthyl-5,5,8,8 naphtyl-2)carbonyl]-4 phénylsulfone,
- Le N-éthyl[(tétrahydro-5,6,7,8 tétraméthyl-5,5,8,8 naphtyl-2) hydroxyméthyl]-4 phénylsulfonamide,
- Le N-éthyl[(tétrahydro-5,6,7,8 tétraméthyl-5,5,8,8 naphtyl-2)carbonyl]-4 phénylsulfonamide,
- La méthyl[E(tétrahydro-5,6,7,8 tétraméthyl-5,5,8,8 naphtyl-2)-3 oxo-3 propényl]-4 phénylsulfone,
- Le méthyl[E(tétrahydro-5,6,7,8 tétraméthyl-5,5,8,8 naphtyl-2)-3 oxo-3 propényl]-4 phénylthioéther,
- Le méthyl[E(tétrahydro-5,6,7,8 tétraméthyl-5,5,8,8 naphtyl-2)-3 oxo-3 propényl]-4 phénylsulfoxyde,
- Le N-éthyl[E(tétrahydro-5,6,7,8 tétraméthyl-5,5,8,8 naphtyl-2)-3 oxo-3 propényl]-4 phénylsulfonamide,
- Le méthyl[E(pentaméthyl-1,1,2,3,3 indanyl-5)-3 oxo-3 propényl]-4 phénylthioéther,
- Le méthyl[E(pentaméthyl-1,1,2,3,3 indanyl-5)-3 oxo-3 propényl]-4 phénylsulfoxyde,
- La méthyl[E(pentaméthyl-1,1,2,3,3 indanyl-5)-3 oxo-3 propényl]-4 phénylsulfone,
- La méthyl[E(pentaméthyl-1,1,2,3,3 indanyl-5)-3 hydroxy-3 propényl]-4 phénylsulfone,
- La méthyl[E(tétrahydro-5,6,7,8 tétraméthyl-5,5,8,8 naphtyl-2)-3 méthoxy-3 propényl]-4 phénylsulfone,
- L'éthyl[(tétrahydro-5,6,7,8 tétraméthyl-5,5,8,8 naphtyl-2)carbonyl]-6 naphtylsulfone-2,
- L'éthyl[(pentaméthyl-1,1,2,3,3 indanyl-5)carbonyl]-6 naphtylsulfone-2,
- L'éthyl[E(tétraméthyl-1,1,3,3 indanyl-5)-3 oxo-3 propényl]-4 phénylsulfone,
- La méthyl[(pentaméthyl-1,1,2,3,3 indanyl-5)-3 méthoxy-3 propényl]-4 phénylsulfone,
- L'éthyl[(tétraméthyl-1,1,3,3 indanyl-5)carbonyl]-6 naphtylsulfone-2,
- La dihydroxy-2′,3′ propyl [(tétrahydro-5,6,7,8 tétraméthyl-5,5,8,8, naphtyl-2)carbonyl]-6 naphtylsulfone-2,
- L'éthyl[(tétrahydro-5,6,7,8 tétraméthyl-5,5,8,8 naphtyl-2) hydroxyméthyl]-6 naphtylsulfone-2,
- L'éthyl[(méthano-5,8 tétrahydro-5,6,7,8 naphtyl-2)carbonyl]-6 naphtylsulfone-2,
- L'éthyl[(méthano-5,8 tétrahydro-5,6,7,8 naphtyl-2)carbonyl]-6 naphtylsulfoxyde-2,
- L'éthyl[(tétrahydro-5,6,7,8 tétraméthyl-5,5,8,8 naphtyl-2)méthylène]-6 naphtylsulfone-2,
- L'éthyl[(tétrahydro-5,6,7,8 tétraméthyl-5,5,8,8 naphtyl-2)-1 hydroxy-1 éthyl naphtylsulfone-2,
- Le N-éthyl[(tétrahydro-5,6,7,8 tétraméthyl-5,5,8,8 naphtyl-2)carbonyl]-4 naphtylsulfonamide-2,
- L'éthyl[(tétrahydro-5,6,7,8 tétraméthyl-5,5,8,8 naphtyl-2)-1 hydroxy-1 éthyl]-6 naphtyl thioéther-2,
- L'éthyl[(tétrahydro-5,6,7,8 tétraméthyl-5,5,8,8 naphtyl-2)-1 éthényl]-6 naphtyl thioéther-2,
- L'ethyl[(tétrahydro-5,6,7,8 tétraméthyl-5,5,8,8 naphtyl-2)carbonyl]-6 naphtylsulfoxyde-2,
- L'éthyl[(diméthyl-5,8 méthoxy-6 naphtyl-2)carbonyl]-6 naphtylsulfone-2,

– L'éthyl[(diméthyl-5,8 méthoxy-6 naphtyl-2)hydroxyméthyl]-6 naphtylsulfone-2, et
– L'éthyl[(diméthyl-5,8 méthoxy-6 naphtyl-2)méthylène]-6 naphtylsulfone-2.

11. Médicament caractérisé par le fait qu'il est un composé de formule (I) selon l'une quelconque des revendications 1 à 10.

12. Composition pharmaceutique caractérisée par le fait qu'elle contient dans un véhicule approprié pour une administration par voie entérale, parentérale, topique ou oculaire, au moins un composé de formule (I) selon l'une quelconque des revendications 1 à 10.

13. Composition selon la revendication 12 caractérisée par le fait qu'elle se présente sous une forme appropriée pour une administration topique ou oculaire et contient de 0,0001 à environ 5%, et de préférence de 0,001 à 1%, en poids d'un composé de formule (I).

14. Utilisation d'un composé selon l'une quelconque des revendications 1 à 10 pour la préparation d'une composition pharmaceutique destinée au traitement des affections dermatologiques, respiratoires ainsi qu'ophtalmologiques.

15. Composition cosmétique pour l'hygiène corporelle et capillaire, caractérisée par le fait qu'elle contient dans un véhicule cosmétique approprié au moins un composé de formule (I) selon l'une quelconque des revendications 1 à 10.

16. Composition cosmétique selon la revendication 15 caractérisée par le fait qu'elle contient le composé de formule (I) à une concentration comprise entre 0,0001 et 2% et de préférence entre 0,001 et 1% en poids.

17. Composition selon les revendications 12, 13, 15 et 16 caractérisée par le fait qu'elle contient un additif pris dans le groupe constitué par les agents hydratants, antiséborrhéiques, anti-acnéiques, les antibiotiques, les agents favorisant la repousse des cheveux, les agents anti-inflammatoires, les caroténoïdes et les agents antipsoriasiques.

**Revendications pour les états contractants : ES, GR**

1. Procédé de préparation de composés polycycliques aromatiques correspondant à la formule générale suivante :

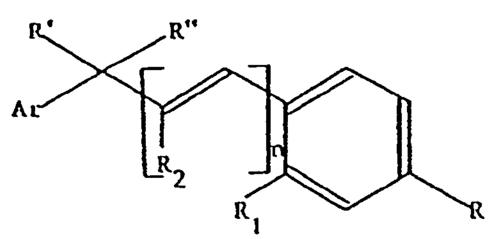

(I)

et leurs isomères géométriques et optiques dans laquelle :

R représente

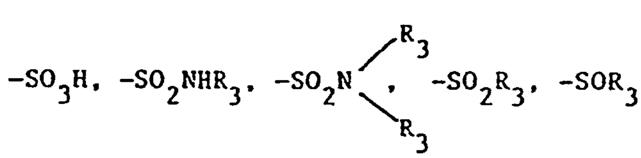

ou –SR3,

$R_3$ représentant un radical alkyle inférieur, linéaire ou ramifié, un radical monohydroxyalkyle, polyhydroxyalkyle, aminoalkyle, carboxyalkyle, aminocarboxyalkyle ou alkoxyalkyle,

R' représente un atome d'hydrogène, un radical alkyle inférieur, un radical alkoxy inférieur, un radical hydroxyle, un radical acyloxy en $C_1$-$C_4$ ou un radical amino,

R" représente un atome d'hydrogène, un radical alkyle inférieur ou un radical alkoxy inférieur, ou

R' et R", pris ensemble, forment un radical oxo (=O), méthano (=$CH_2$)ou hydroxyimino (=N–OH),

$R_1$ représente un atome d'hydrogène ou un radical alkyle inférieur, n est 0 ou 1,
lorsque n=1, $R_2$ représente un atome d'hydrogène ou un radical alkyle inférieur, ou $R_1$ et $R_2$, pris ensemble, forment un radical vinylène (–CH=CH–), et

Ar représente un radical aromatique selon l'une des formules suivantes :

(i)

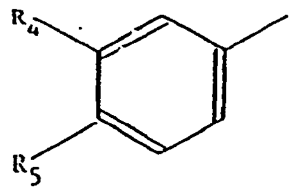

(ii)

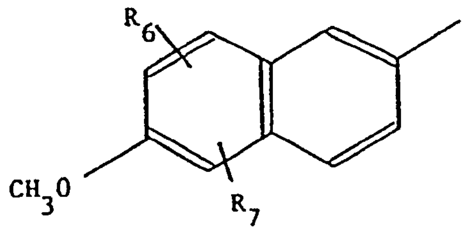

(iii)

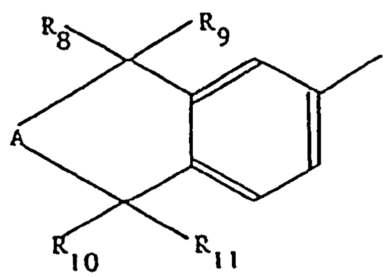

dans lesquelles :

$R_4$, $R_5$, $R_6$, $R_7$, $R_8$, $R_9$, $R_{10}$, et $R_{11}$ représentent un atome d'hydrogène ou un radical alkyle inférieur, au moins un des radicaux $R_4$ et/ou $R_5$ étant différent d'un atome d'hydrogène, et au moins deux des radicaux $R_8$ à $R_{11}$ étant différents d'un atome d'hydrogène,

A représente un radical méthylène ou diméthylène substitué ou non par un radical alkyle inférieur ; lorsque A représente un radical diméthylène, $R_8$ et $R_{10}$ peuvent former ensemble un radical méthylène ou diméthylène, caractérisé par le fait qu'il consiste à acyler dans les conditions de la réaction de Friedel-Crafts un composé aromatique de formule : ArH dans laquelle Ar à la même signification que ci-dessus, par un chlorure d'acide benzénique ou naphtalénique de formule :

Ar+ 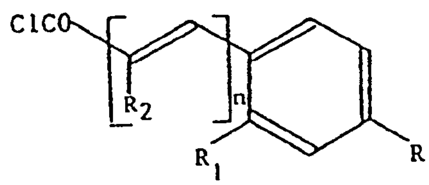

dans laquelle :

n, R, $R_1$ et $R_2$ ont les mêmes significations que ci-dessus, et à soumettre éventuellement le composé carbonylé obtenu aux réactions subséquentes permettant d'accéder aux autres significations des radicaux R', R" et R.

2. Procédé selon la revendication 1, caractérisé par le fait que le composé carbonylé obtenu est soumis à une réaction de réduction en présence de borohydrure de sodium dans un solvant organique en vue d'obtenir le composé de formule (I) dans laquelle R' = OH et R" = H.

3. Procédé selon la revendication 1, caractérisé par le fait que le composé carbonylé obtenu est soumis à une réaction de réduction au zinc dans l'acide acétique en vue d'obtenir le composé de formule (I) dans laquelle R' =R" = H.

4. Procédé selon la revendication 2, caractérisé par le fait que le composé hydroxylé obtenu est acylé à l'aide d'un anhydride ou d'un chlorure d'acide en vue d'obtenir le composé de formule (I) dans laquelle

R′ = acyloxy et R″ = H.

5. Procédé selon la revendication 2, caractérisé par le fait que le composé hydroxylé obtenu est éthérifié selon les méthodes connues en vue d'obtenir le composé de formule (I) dans laquelle R′= alkoxy et R″ = H.

6. Procédé selon la revendication 1, caractérisé par le fait que le composé carbonylé est soumis à une réaction de Wittig en présence de bromure de triphényl méthyl phosphonium et d'amidure de sodium en vue d'obtenir le composé de formule (I) dans laquelle R′ et R″, pris ensemble, forment le radical méthano (CH2=).

7. Procédé selon l'une quelconque des revendications précédentes, caractérisé par le fait que les composés obtenus de formule (I) ayant une fonction acide libre sont transformés en sel d'un métal alcalin ou alcalino-terreux ou de zinc ou d'une amine organique.

8. Procédé selon l'une quelconque des revendications 1 à 6, caractérisé par le fait que les composés obtenus de formule (I) ayant une fonction amine libre sont transformés en sel d'un acide minéral ou organique,de préférence l'acide chlorhydrique, bromhydrique ou citrique.

9. Composition cosmétique pour l'hygiène corporelle et capillaire, caractérisée par le fait qu'elle contient dans un véhicule cosmétique approprié au moins un composé de formule (I) obtenu selon l'une quelconque des revendications 1 à 8.

10. Composition cosmétique selon la revendications 9, caractérisée par le fait qu'elle contient le composé de formule (I) à une concentration comprise entre 0,0001 et 2% et de préférence entre 0,001 et 1% en poids.

11. Composition selon les revendications 9 et 10, caractérisée par le fait qu'elle contient un additif pris dans le groupe constitué par les agents hydratants, antiséborrhéiques, anti-acnéiques, les antibiotiques, les agents favorisant la repousse des cheveux, les agents anti-inflammatoires, les caroténoides et les agents antipsoriasiques.

## Claims

### Claims For the Contracting States : ES, GR

1. Process for the preparation of aromatic polycyclic compounds corresponding to the following general formula :

R represents

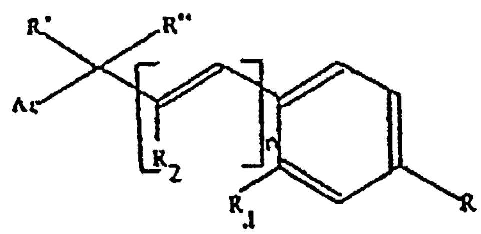

(I)

and their geometrical and optical isomers, in which :

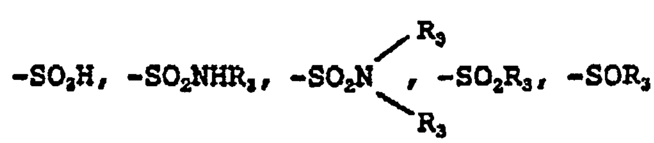

or $-SR_3$,

$R_3$ representing a straight-chain or branched lower alkyl radical or a monohydroxyalkyl, polyhydroxyalkyl, aminoalkyl, carboxyalkyl, aminocarboxyalkyl or alkoxyalkyl radical,

R′ represents a hydrogen atom, a lower alkyl radical, a lower alkoxy radical, a hydroxyl radical, a $C_1$-$C_4$ acyloxy radical or an amino radical,

R″ represents a hydrogen atom, a lower alkyl radical or a lower alkoxy radical, or

R′ and R″, taken together, form an oxo (=O), methano (=$CH_2$) or hydroxyimino (=N–OH) radical,

$R_1$ represents a hydrogen atom or a lower alkyl radical, and

n is 0 or 1,

when n=1, $R_2$ represents a hydrogen atom or a lower alkyl radical, or $R_1$ and $R_2$, taken together, form a vinylene radical (–CH=CH–), and

Ar represents an aromatic radical according to one of the following formulae :

(i)

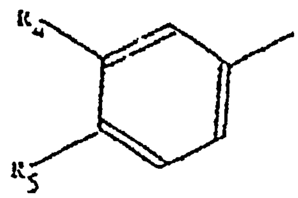

(ii)

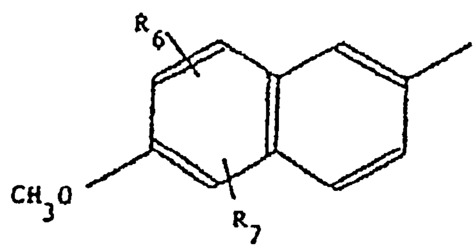

(iii)

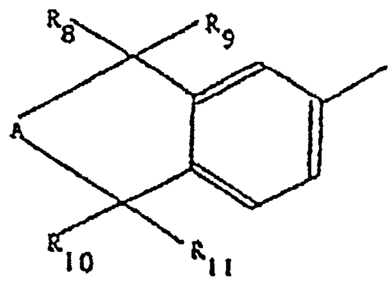

in which,

$R_4$, $R_5$, $R_6$, $R_7$, $R_8$, $R_9$, $R_{10}$ and $R_{11}$ represent a hydrogen atom or a lower alkyl radical, at least one of the radicals $R_4$ and/or $R_5$ differing from a hydrogen atom and at least two of the radicals $R_8$ to $R_{11}$ differing from a hydrogen atom, and

A represents a methylene or dimethylene radical which is unsubstituted or substituted by a lower alkyl radical ; when A represents a dimethylene radical, $R_8$ and $R_{10}$ may together form a methylene or dimethylene radical, characterized in that it consists in acylating an aromatic compound of formula : ArH, in which Ar has the same meaning as above, under the conditions for the Friedel-Crafts reaction, with a benzene-based or napthalene-based acid chloride of formula :

Ar +

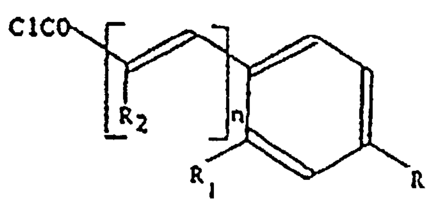

in which :

n, R, $R_1$ and $R_2$ have the same meanings as above, and, if appropriate, subjecting the carbonyl compound obtained to the subsequent reactions enabling the other meanings of the radicals R′, R″ and R to be obtained.

2. Process according to Claim 1, characterized in that the carbonyl compound obtained is subjected to a reduction reaction in the presence of sodium borohydride in an organic solvent with a view to obtaining

the compound of formula (I) in which R′ = OH and R″ = H.

3. Process according to Claim 1, characterized in that the carbonyl compound obtained is subjected to a reduction reaction with zinc in acetic acid with a view to obtaining the compound of formula (I) in which R′ = R″ = H.

4. Process according to Claim 2, characterized in that the hydroxyl compound obtained is acylated using an acid anhydride or acid chloride with a view to obtaining the compound of formula (I) in which R′ = acyloxy and R″ = H.

5. Process according to Claim 2, characterized in that the hydroxyl compound obtained is etherified by the known methods with a view to obtaining the compound of formula (I) in which R′ = alkoxy and R″ = H.

6. Process according to Claim 1, characterized in that the carbonyl compound is subjected to a Wittig reaction in the presence of triphenylmethylphosphonium bromide and sodium amide with a view to obtaining the compound of formula (I) in which R′ and R″, taken together, form the methano ($CH_2$=) radical.

7. Process according to any one of the preceding claims, characterized in that the compounds obtained of formula (I) having a free acid function are converted to an alkali metal or alkaline earth metal salt or a zinc salt or an organic amine salt.

8. Process according to any one of Claims 1 to 6, characterized in that the compounds obtained of formula (I) having a free amine function are converted to a salt of an inorganic or organic acid, preferably hydrochloric, hydrobromic or citric acid.

9. Cosmetic composition for bodily and hair hygiene, characterized in that it contains at least one compound of formula (I) obtained according to any one of Claims 1 to 8 in a suitable cosmetic vehicle.

10. Cosmetic composition according to Claim 9, characterized in that it contains the compound of formula (I) in a concentration of between 0.0001 and 2% and preferably between 0.001 and 1% by weight.

11. Composition according to Claims 9 and 10, characterized in that it contains an additive taken from the group comprising hydrating, antiseborrheic and antiacne agents, antibiotics, agents promoting hair growth, anti-inflammatory agents, carotenoids and antipsoriasis agents,

**Claims For the Contracting States : AT, BE, CH, DE, FR, GB, IT, LI, NL, SE**

1. Aromatic polycyclic compounds, characterized in that they correspond to the following formula :

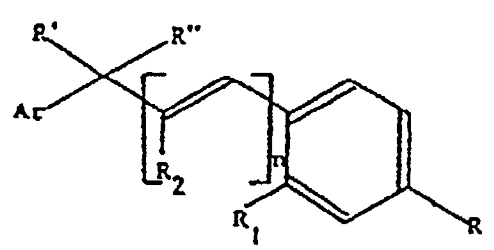

(I)

in which :

R represents

$-SO_3H$, $-SO_2NHR_3$, 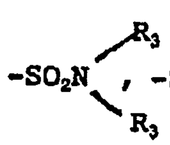, $-SO_2R_3$, $-SOR_3$

or $-SR_3$,

$R_3$ representing a straight-chain or branched lower alkyl radical or a monohydroxyalkyl, polyhydroxyalkyl, aminoalkyl, carboxyalkyl, aminocarboxyalkyl or alkoxyalkyl radical,

R′ represents a hydrogen atom, a lower alkyl radical, a lower alkoxy radical, a hydroxyl radical, a $C_1$-$C_4$ acyloxy radical or an amino radical

R″ represents a hydrogen atom, a lower alkyl radical or a lower alkoxy radical, or

R′ and R″, taken together, form an oxo (=O), methano (=$CH_2$) or hydroxyimino (=N–OH) radical

$R_1$ represents a hydrogen atom or a lower alkyl radical, and

n is 0 or 1,

when n=1, $R_2$ represents a hydrogen atom or a lower alkyl radical, or $R_1$ and $R_2$, taken together, form a vinylene radical (–CH=CH–), and

Ar represents an aromatic radical according to one of the following formulae :

(i)

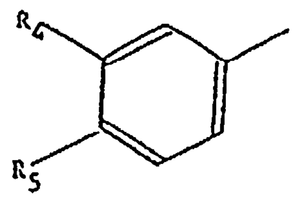

(ii)

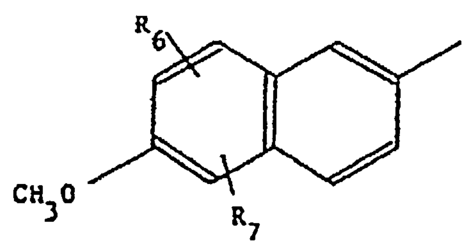

(iii)

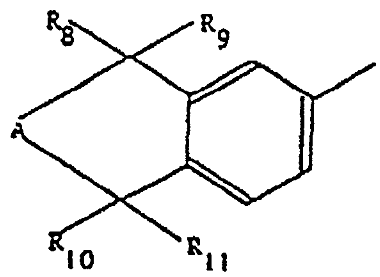

in which :

$R_4$, $R_5$, $R_6$, $R_7$, $R_8$, $R_9$, $R_{10}$ and $R_{11}$ represent a hydrogen atom or a lower alkyl radical, at least one of the radicals $R_4$ and/or $R_5$ differing from a hydrogen atom and at least two of the radicals $R_8$ to $R_{11}$ differing from a hydrogen atom, and

A represents a methylene or dimethylene radical which is unsubstituted or substituted by a lower alkyl radical ; when A represents a dimethylene radical, $R_8$ and $R_{10}$ may together form a methylene or dimethylene radical,

and the salts of the said compounds of formula (I) as well as their geometrical and optical isomers.

2. Compounds according to Claim 1, characterized in that the lower alkyl radical is taken from the group comprising the methyl, ethyl, isopropyl, butyl and tert-butyl radicals.

3. Compounds according to Claim 1, characterized in that the monohydroxyalkyl radical is a radical having from 2 to 6 carbon atoms, in particular the 2-hydroxy ethyl radical or the 2 -hydroxy propyl radical.

4. Compounds according to Claim 1, characterized in that the polyhydroxyalkyl radical is a radical having from 3 to 6 carbon atoms and from 2 to 5 hydroxyl groups, in particular the 2,3-dihydroxypropyl or 1,3-dihydroxy-2-propyl radical or the radical of pentaerythritol.

5. Compounds according to Claim 1, characterized in that the aminoalkyl radical comprises from 2 to 6 carbon atoms, such as the 2-aminoethyl, 2-aminopropyl or 3-aminopropyl radicals.

6. Compounds according to Claim 1, characterized in that the carboxyalkyl radical comprises from 2 to 7 carbon atoms, such as the carboxymethyl, 2-carboxyethyl, 2-carboxypropyl or 3-carboxybutyl radicals.

7. Compounds according to Claim 1, characterized in that the aminocarboxyalkyl radical is the 2-amino-2-carboxyethyl or 3-amino-3-carboxypropyl radical.

8. Compounds according to Claim 1, characterized in that the lower alkoxy radical is the methoxy, ethoxy, butoxy or isopropoxy radical.

9. Compounds according to any one of Claims 1 to 8, characterized in that they correspond to the following formula :

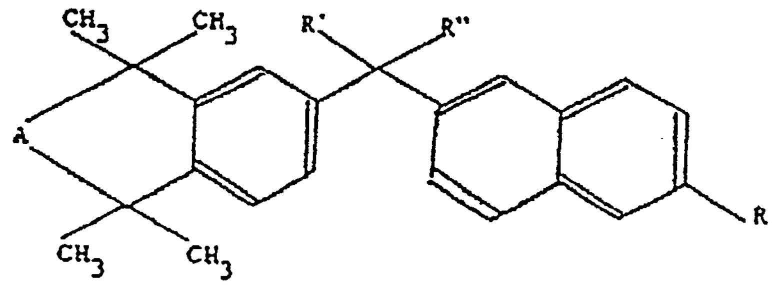

(II)

in which :

R represents $-SO_2R'_3$, $SO_2NHR'_3$, $SOR'_3$ or $-SR'_3$,

$R'_3$ representing a lower alkyl radical,

R' represents a hydrogen atom or a hydroxyl or lower alkyl radical, and

R" represents a hydrogen atom, or R' and R" taken together form an oxo (=O) radical, and

A represents a dimethylene radical or a radical of formula

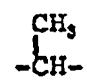

10. Compounds according to any one of Claims 1 to 9, characterized in that they are taken from the group comprising :

- methyl 4-[(5,6,7,8-tetrahydro-5,5,8,8-tetramethyl-2-naphthyl)hydroxymethyl]-phenyl sulfone,
- methyl 4-[(5,6,7,8-tetrahydro-5,5,8,8-tetramethyl-2-naphthyl)carbonyl]-phenyl sulfone,
- N-ethyl 4-[(5,6,7,8-tetrahydro-5,5,8,8-tetramethyl-2-naphthyl)hydroxymethyl]-phenyl sulfonamide,
- N-ethyl 4-[(5,6,7,8-tetrahydro-5,5,8,8-tetramethyl-2-naphthyl)carbonyl]-phenyl sulfonamide,
- methyl 4-[3-E-(5,6,7,8-tetrahydro-5,5,8,8-tetramethyl-2-naphthyl)-3-oxo-propenyl]-phenyl sulfone,
- methyl 4-[3-E-(5,6,7,8-tetrahydro-5,5,8,8-tetramethyl-2-naphthyl)-3-oxopropenyl]-phenyl thioether,
- methyl 4-[3-E-(5,6,7,8-tetrahydro-5,5,8,8-tetramethyl-2-naphthyl)-3-oxopropenyl]-phenyl sulfoxide,
- N-ethyl 4-[3-E-(5,6,7,8-tetrahydro-5,5,8,8-tetramethyl-2-naphthyl)-3-oxopropenyl]-phenyl sulfonamide,
- methyl 4-[3-E-(1,1,2,3,3-pentamethyl-5-indanyl)-3-oxopropenyl]-phenyl thioether,
- methyl 4-[3-E-(1,1,2,3,3-pentamethyl-5-indanyl)-3-oxopropenyl]-phenyl sulfoxide,
- methyl 4-[3-E-(1,1,2,3,3-pentamethyl-5-indanyl)-3-oxopropenyl]-phenyl sulfone,
- methyl 4-[3-E-(1,1,2,3,3-pentamethyl-5-indanyl)-3-hydroxypropenyl]-phenyl sulfone,
- methyl 4-[3-E-(5,6,7,8-tetrahydro-5,5,8,8-tetramethyl-2-naphthyl)-3-methoxypropenyl]-phenyl sulfone,
- ethyl 6-[(5,6,7,8-tetrahydro-5,5,8,8-tetramethyl-2-naphthyl)carbonyl]-2-naphthyl sulfone,
- ethyl 6-[(1,1,2,3,3-pentamethyl-5-indanyl)carbonyl]-2-naphthyl sulfone,
- ethyl 4-[3-E-(1,1,3,3-tetramethyl-5-indanyl)-3-oxopropenyl]-phenyl sulfone,
- methyl 4-[3-(1,1,2,3,3-pentamethyl-5-indanyl)-3-methoxypropenyl]-phenyl sulfone,
- ethyl 6-[(1,1,3,3-tetramethyl-5-indanyl)-carbonyl]-2-naphthyl sulfone,
- 2',3'- Dihydroxypropyl 6-[(5,6,7,8-tetrahydro-5,5,8,8-tetramethyl-2-naphthyl)carbonyl]-2-naphthyl sulfone,
- ethyl 6-[(5,6,7,8-tetrahydro-5,5,8,8-tetramethyl-2-naphthyl)hydroxymethyl]-2-naphthyl sulfone,
- ethyl 6-[(5,8-methano-5,6,7,8-tetrahydro-2-naphthyl)-carbonyl]-2-naphthyl sulfone,
- ethyl 6-[(5,8-methano-5,6,7,8-tetrahydro-2-naphthyl)-carbonyl]-2-naphthyl sulfoxide,
- ethyl 6-[(5,6,7,8-tetrahydro-5,5,8,8-tetramethyl-2-naphthyl)-methylene]-2-naphthyl sulfone,
- ethyl 6-[1-(5,6,7,8-tetrahydro-5,5,8,8-tetramethyl-2-naphthyl)-1-hydroxyethyl]-2-naphthyl sulfone,
- N-ethyl 4-[(5,6,7,8-tetrahydro-5,5,8,8-tetramethyl-2-naphthyl)carbonyl]-2-naphthyl sulfonamide,
- ethyl 6-[-(5,6,7,8-tetrahydro-5,5,8,8-tetramethyl-2-naphthyl)-1-hydroxyethyl]-2-naphthyl thioether,
- ethyl 6-[-(5,6,7,8-tetrahydro-5,5,8,8-tetramethyl-2-naphthyl)ethenyl]-2-naphthyl thioether,
- ethyl 6-[(5,6,7,8-tetrahydro-5,5,8,8-tetramethyl-2-naphthyl)carbonyl]-2-naphthyl sulfoxide,
- ethyl 6-[(5,8-dimethyl-6-methoxy-2-naphthyl)carbonyl]-2-naphthyl sulfone,
- ethyl 6-[(5,8-dimethyl-6-methoxy-2-naphthyl)hydroxymethyl]-2-naphthyl sulfone, and
- ethyl 6-[(5,8-dimethyl-6-methoxy-2-naphthyl)methylene] -2-naphthyl sulfone.

11. Medicament, characterized in that it is a compound of formula (I) according to any one of Claims 1 to 10.

12. Pharmaceutical composition, characterized in that it contains at least one compound of formula (I) according to any one of Claims 1 to 10 in a vehicle suitable for enteral or parenteral administration or topical or application ocular.

13. Composition according to Claim 12, characterized in that it is in a form suitable for topical or ocular application and contains from 0.0001 to about 5%, and preferably from 0.001 to 1%, by weight of a compound of formula (r).

14. Use of a compound according to any one of Claims 1 to 10 for the preparation of a pharmaceutical composition intended for the treatment of dermatological, respiratory and ophthalmological diseases.

15. Cosmetic composition for bodily and hair hygiene, characterized in that it contains at least one compound of formula (I) according to any one of Claims 1 to 10 in a suitable cosmetic vehicle.

16. Cosmetic composition according to Claim 15, characterized in that it contains the compound of formula (I) in a concentration of between 0.0001 and 2% and preferably between 0. 001 and 1% by weight.

17. Composition according to Claims 12, 13, 15, and 16, characterized in that it contains an additive taken from the group comprising hydrating, antiseborrheic and anti-acne agents, antibiotics, agents promoting hair growth, anti-inflammatory agents, carotenoids and antipsoriasis agents.

## Ansprüche

### Patentansprüche für die Vertragsstaaten ES, GR

1. Verfahren zur Herstellung polycyclisch aromatischer Verbindungen folgender Formel :

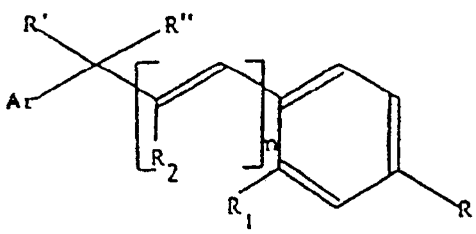

(I)

worin

R für

$-SO_3H$, $-SO_2NHR_3$, 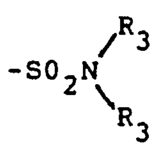 , $-SO_2R_3$, $-SOR_3$ oder

$-SR_3$ steht,

wobei $R_3$ für einen geraden oder verzweigten Niedrigalkylrest, einen Monohydroxyalkyl-, Polyhydroxyalkyl-, Aminoalkyl-, Carboxyalkyl-, Aminocarboxyalkyl- oder Alkoxyalkylrest steht,

R′ für ein Wasserstoffatom, einen Niedrigalkylrest, einen Niedrigalkoxyrest, einen Hydroxylrest, einen $C_1$-$C_4$-Acyloxyrest oder einen Aminorest steht,

R″ für ein Wasserstoffatom, einen Niedrigalkylrest oder einen Niedrigalkoxyrest steht, oder

R′ und R″ zusammen für einen Oxo-(=O), Methano-(=$CH_2$) oder Hydroxyimino-(=N–OH) Rest stehen,

$R_1$ für ein Wasserstoffatom oder einen Niedrigalkylrest steht,

n für 0 oder 1 steht,

wenn n = 1 ist,

$R_2$ für ein Wasserstoffatom oder einen Niedrigalkylrest steht, oder

$R_1$ und $R_2$ zusammen einen Vinylen-(–CH=CH–) Rest bilden, und Ar für einen aromatischen Rest folgender Formeln steht :

(i)

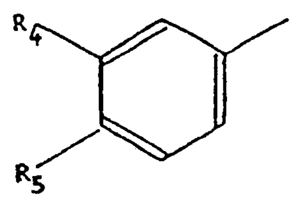

(ii)

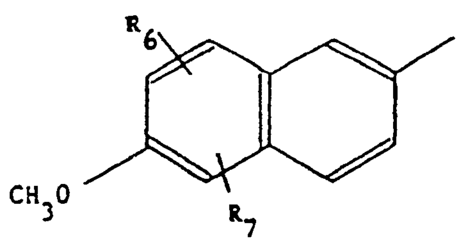

(iii)

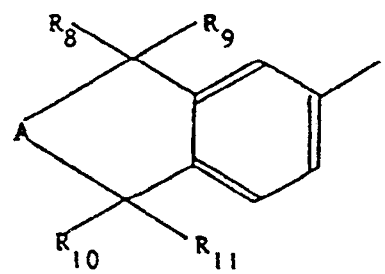

worin $R_4$, $R_5$, $R_6$, $R_7$, $R_8$, $R_9$, $R_{10}$ und $R_{11}$ für ein Wasserstoffatom oder einen Niedrigalkylrest stehen, wobei mindestens einer der Reste $R_4$ und/oder $R_5$ nicht für Wasserstoff steht und mindestens zwei der Reste $R_8$ bis $R_{11}$ nicht für Wasserstoff stehen,

A für einen ggf. mit einem Niedrigalkylrest substituierten Methylen- oder Dimethylenrest steht, und wenn A für einen Dimethylenrest steht, $R_8$ und $R_{10}$ zusammen einen Methylen- oder Dimethylenrest bilden können, **dadurch gekennzeichnet**, daß man unter den Bedingungen einer Friedel-Craft-Reaktion eine aromatische Verbindung der Formel ArH, worin

Ar die oben angegebenen Bedeutungen besitzt, mit einem Benzoesäure- oder Naphthoesäurechlorid der Formel :

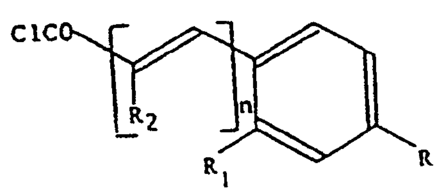

worin n, R, $R_1$ und $R_2$ die oben angegebenen Bedeutungen besitzen, acyliert und die erhaltene Carbonylverbindung gegebenenfalls weiteren Reaktionen unterwirft, die einen Zugang zu den anderen Bedeutungen der Reste R', R" und R ermöglichen.

2. Verfahren nach Anspruch 1, dadurch gekennzeichnet, daß die erhaltene Carbonylverbindung einer Reduktion in Anwesenheit von Natriumborhydrid in einem organischen Lösungsmittel unterworfen wird, um eine Verbindung der Formel I, worin R' = OH und R" = H, zu erhalten.

3. Verfahren nach Anspruch 1, dadurch gekennzeichnet, daß die erhaltene Carbonylverbindung einer Reduktion mit Zink in Essigsäure unterworfen wird,um eine Verbindung der Formel (I), worin R′ = R″ = H, zu erhalten.

4. Verfahren nach Anspruch 2, dadurch gekennzeichnet, daß die erhaltene Hydroxyverbindung mit einem Säureanhydrid oder Säurechlorid acyliert wird, um eine Verbindung der Formel (I), worin R′ = Acyloxy und R″ = H, zu erhalten.

5. Verfahren nach Anspruch 2, dadurch gekennzeichnet, daß die erhaltene Hydroxyverbindung nach bekannten Verfahren veräthert wird, um eine Verbindung der Formel (I), worin R′ = Alkoxy und R″ = H, zu erhalten.

6. Verfahren nach Anspruch 1, dadurch gekennzeichnet, daß die Carbonylverbindung einer Wittig-Reaktion in Anwesenheit von Triphenylmethylphosphoniumbromid und Natriumamid unterworfen wird, um eine Verbindung der Formel (I), worin R′ und R″ zusammen für den Methano-($CH_2$=) Rest stehen, zu erhalten.

7. Verfahren nach einem der vorhergehenden Ansprüche, dadurch gekennzeichnet, daß die erhaltenen Verbindungen der Formel (I) mit einer freien Säurefunktion in ein Alkalimetall- oder Erdalkalimetall- oder Zinksalz oder in ein Salz mit einem organischen Amin überführt werden.

8. Verfahren nach einem der Ansprüche 1 bis 6, dadurch gekennzeichnet, daß die erhaltenen Verbindungen der Formel (I) mit einer freien Aminfunktion in ein Salz einer Mineralsäure oder einer organischen Säure, vorzugsweise Chlorwasserstoffsäure, Bromwasserstoffsäure oder Citronensäure, überführt werden.

9. Kosmetisches Mittel zur Hygiene des Körpers und der Haare, dadurch gekennzeichnet, daß es in einem geeigneten kosmetischen Träger wenigstens eine Verbindung der Formel (I), die nach einem der Ansprüche 1 bis 8 erhalten wurde, enthält.

10. Kosmetisches Mittel nach Anspruch 9, dadurch gekennzeichnet, daß es die Verbindung der Formel (I) in einer Konzentration zwischen 0,0001 und 2 Gew.-% und vorzugsweise zwischen 0,001 und 1 Gew.-%, enthält.

11. Mittel nach den Ansprüchen 9 und 10, dadurch gekennzeichnet, daß es ein Additiv enthält, das ausgewählt ist unter Hydratisierungsmitteln, Antiseborrhoe-Mitteln, Antiakne-Mitteln, Antibiotika, Haarwuchsmitteln, anti-inflammatorischen Mitteln, Carotinoiden und Antipsoriasis-Mitteln.

**Patentansprüche für die Vertragsstaaten AT, BE, CH, DE, FR, GB, IT, LI, NL, SE**

1. Polycyclische aromatische Verbindungen folgender Formel :

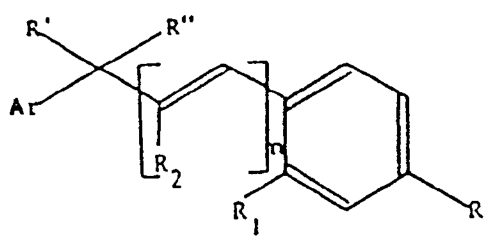

(I)

worin

R für

$-SO_3H$, $-SO_2NHR_3$, 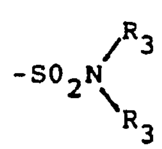 , $-SO_2R_3$, $-SOR_3$ oder

$-SR_3$ steht,

wobei $R_3$ für einen geraden oder verzweigten Niedrigalkylrest, einen Monohydroxyalkyl-, Polyhydroxyalkyl-, Aminoalkyl-, Carboxyalkyl-, Aminocarboxyalkyl- oder Alkoxyalkylrest steht,

R′ für ein Wasserstoffatom, einen Niedrigalkylrest, einen Niedrigalkoxyrest, einen Hydroxylrest, einen $C_1$-$C_4$ Acyloxyrest oder einen Aminorest steht,

R″ für ein Wasserstoffatom, einen Niedrigalkylrest oder einen Niedrigalkoxyrest steht, oder

R′ und R″ zusammen für einen Oxo-(=O), Methano-(=$CH_2$) oder Hydroxyimino-(=N–OH) Rest stehen,

$R_1$ für ein Wasserstoffatom oder einen Niedrigalkylrest steht,
n für 0 oder 1 steht,
wenn n = 1 ist,
$R_2$ für ein Wasserstoffatom oder einen Niedrigalkylrest steht, oder
$R_1$ und $R_2$ zusammen einen Vinylen-(–CH=CH–) Rest bilden, und
Ar für einen aromatischen Rest folgender Formeln steht :

(i)

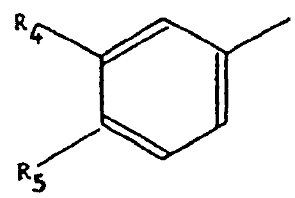

(ii)

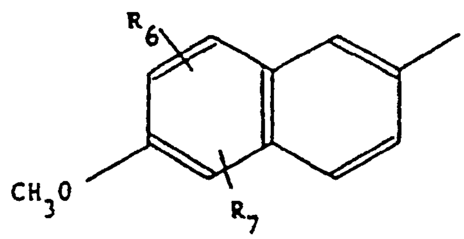

(iii)

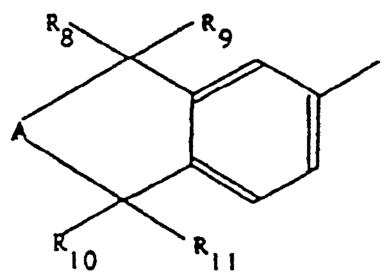

worin $R_4$, $R_5$, $R_6$, $R_7$, $R_8$, $R_9$, $R_{10}$ und $R_{11}$ für ein Wasserstoffatom oder einen Niedrigalkylrest stehen, wobei mindestens einer der Reste $R_4$ und/oder $R_5$ nicht für Wasserstoff steht und mindestens zwei der Reste $R_8$ bis $R_{11}$ nicht für Wasserstoff stehen,

A für einen ggf. mit einem Niedrigalkylrest substituierten Methylen- oder Dimethylenrest steht, und wenn A für einen Dimethylenrest steht, $R_8$ und $R_{10}$ zusammen einen Methylen- oder Dimethylenrest bilden können,

und die Salze der Verbindungen der Formel (I) sowie ihre geometrischen und optischen Isomere.

2. Verbindungen nach Anspruch 1, **dadurch gekennzeichnet**, daß der Niedrigalkylrest ausgewählt ist unter einem Methyl-, Ethyl-, i-Propyl-, Butyl-und t-Butylrest.

3. Verbindungen nach Anspruch 1, **dadurch gekennzeichnet**, daß der Monohydroxyalkylrest ein Rest mit 2 bis 6 Kohlenstoffatomen, insbesondere ein 2-Hydroxy-ethyl- oder 2-Hydroxypropylrest, ist.

4. Verbindungen nach Anspruch 1, **dadurch gekennzeichnet**, daß der Polyhydroxyalkylrest ein Rest mit 3 bis 6 Kohlenstoffatomen und 2 bis 5 Hydroxylgruppen, insbesondere ein 2,3-Dihydroxypropyl-, 1,3-Dihydroxy-2-propyl- oder Pentaerythritrest, ist.

5. Verbindungen nach Anspruch 1, **dadurch gekennzeichnet**, daß der Aminoalkylrest 2 bis 6 Kohlenstoffatome umfaßt, wie de 2-Aminoethyl-, 2-Amino-propyl- oder 3-Aminopropylrest.

6. Verbindungen nach Anspruch 1, **dadurch gekennzeichnet**, daß der Carboxyalkylrest 2 bis 7 Kohlenstoffatome umfaßt, wie der Carboxymethyl-, 2-Carboxyethyl-, 2-Carboxypropyl- oder 3-Carboxybutylrest.

7. Verbindungen nach Anspruch 1, **dadurch gekennzeichnet**, daß es sich bei dem Aminocarboxyalkylrest um den 2-Amino-2-carboxyethyl- oder 3-Amino-3-carboxypropylrest handelt.

8. Verbindungen nach Anspruch 1, **dadurch gekennzeichnet**, daß es sich bei dem Niedrigalkoxyrest um den Methoxy-, Ethoxy-, Butoxy- oder i-Propoxyrest handelt.

9. Verbindungen nach einem der Ansprüche 1 bis 8 folgender Formel :

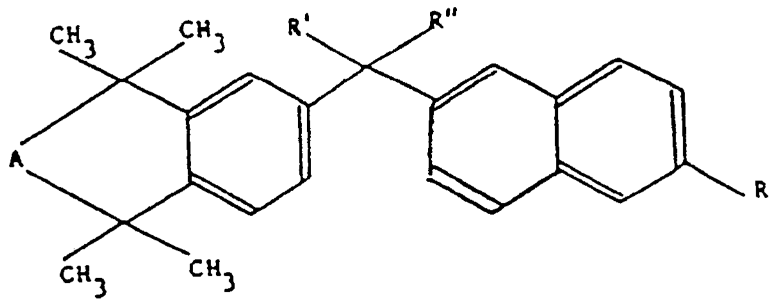

(II)

worin

R für $-SO_2R'_3$, $SO_2NHR'_3$, $SOR'_3$ oder $SR'_3$ steht,

$R'_3$ für einen Niedrigalkylrest steht,

R' für ein Wasserstoffatom, einen Hydroxylrest oder Niedrigalkylrest steht,

R" für ein Wasserstoffatom steht, oder

R' und R" zusammen einen Oxo-(=O) Rest bilden, und A für einen Dimethylenrest oder einen Rest der Formel

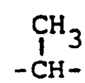

steht.

10. Verbindungen nach einem der Ansprüche 1 bis 9, nämlich :

- Methyl-4-[(5,6,7,8-tetrahydro-5,5,8,8-tetramethyl-2-naphthyl)hydroxymethyl]-phenylsulfon,
- Methyl-4-[(5,6,7,8-tetrahydro-5,5,8,8-tetramethyl-2-naphthyl)carbonyl]-phenylsulfon,
- N-Ethyl-4-[(5,6,7,8-tetrahydro-5,5,8,8-tetramethyl-2-naphthyl)hydroxymethyl]phenylsulfonamid,
- N-Ethyl-4-[(5,6,7,8-tetrahydro-5,5,8,8-tetramethyl-2-naphthyl)carbonyl]-phenylsulfonamid,
- Methyl-4-[E-3(5,6,7,8-tetrahydro-5,5,8,8-tetramethyl-2-naphthyl)-3-oxopropenyl]-phenylsulfon,
- Methyl-4-[E-3(5,6,7,8-tetrahydro-5,5,8,8-tetramethyl-2-naphthyl)-3-oxopropenyl]-phenylthioäther,
- Methyl-4-[E-3(5,6,7,8-tetrahydro-5,5,8,8-tetramethyl-2-naphthyl)-3-oxopropenyl]-phenylsulfoxid,
- N-ethyl-4-[E-3(5,6,7,8-tetrahydro-5,5,8,8-tetramethyl-2-naphthyl)-3-oxopropenyl]-phenylsulfonamid,
- Methyl-4-[E-3(1,1,2,3,3-pentamethyl-5-indanyl)-3-oxopropenyl]-phenylthioäther,
- Methyl-4-[E-3(1,1,2,3,3-pentamethyl-5-indanyl)-3-oxopropenyl]-phenylsulfoxid,
- Methyl-4-[E-3(1,1,2,3,3-pentamethyl-5-indanyl)-3-oxopropenyl]-phenylsulfon,
- Methyl-4-[E-3(1,1,2,3,3-pentamethyl-5-indanyl)-3-hydroxypropenyl]-phenylsulfon,
- Methyl-4-[E-3(5,6,7,8-tetrahydro-5,5,8,8-tetramethyl-2-naphthyl)-3-methoxypropenyl]-phenylsulfon,
- Ethyl- 6-[(5,6,7,8-tetrahydro-5,5,8,8-tetramethyl-2-naphthyl)carbonyl]-2-naphthylsulfon,
- Ethyl- 6-[(1,1,2,3,3-pentamethyl-5-indanyl)carbonyl-2-naphthylsulfon,
- Ethyl-4-[E-3(1,1,3,3-tetramethyl-5-indanyl)-3-oxopropenyl]-phenylsulfon,
- Methyl-4-[3(1,1,2,3,3-pentamethyl-5-indanyl)-3-methoxypropenyl]-phenylsulfon,
- Ethyl-6-[(1,1,3,3-tetramethyl-5-indanyl)carbonyl]-2-naphthylsulfon,
- 2',3'-Dihydroxy-propyl-6-[(5,6,7,8-tetrahydro-5,5,8,8-tetramethyl-2-naphthyl)carbonyl]-2-naphthyl sulfon,
- Ethyl-6-[(5,6,7,8-tetrahydro-5,5,8,8-tetramethyl-2-naphthyl)hydroxymethyl]-2-naphthylsulfon,
- Ethyl- 6-[(5,8-methano-5,6,7,8-tetrahydro-2-naphthyl)-carbonyl]-2-naphthylsulfon,
- Ethyl- 6-[(5,8-methano-5,6,7,8-tetrahydro-2-naphthyl)-carbonyl]-2-naphthylsulfoxid,

- Ethyl- 6-[(5,6,7,8-tetrahydro-5,5,8,8-tetramethyl-2-naphthyl)-methylen]-2-naphthylsulfon,
- Ethyl-[1-[5,6,7,8-tetrahydro-5,5,8,8-tetramethyl-2-naphthyl)-1-hydroxyethyl]-2-naphthylsulfon,
- N-Ethyl- 4-[(5,6,7,8-tetrahydro-5,5,8,8-tetramethyl-2-naphthyl)carbonyl]-2-naphthylsulfonamid,
- Ethyl- 6-[1(5,6,7,8-tetrahydro-5,5,8,8-tetramethyl-2-naphthyl)-1-hydroxyethyl]-2-naphthylthioäther,
- Ethyl-6-[1(5,6,7,8-tetrahydro-5,5,8,8-tetramethyl-2-naphthyl)-ethenyl]-2-naphthylthioäther,
- Ethyl-6-[(5,6,7,8-tetrahydro-5,5,8,8-tetramethyl-2-naphthyl)-carbonyl]-2-naphthylsulfoxid,
- Ethyl- 6-[(5,8-dimethyl-6-methoxy-2-naphthyl)-carbonyl]-2-naphthylsulfon,
- Ethyl- 6-[(5,8-dimethyl-6-methoxy-2-naphthyl)-hydroxymethyl]-2-naphthylsulfon und
- Ethyl- 6-[(5,8-dimethyl-6-methoxy-2-naphthyl)-methylen]-2-naphthylsulfon.

11. Arzneimittel, **dadurch gekennzeichnet** daß es sich dabei um eine Verbindung der Formel (I) nach einem der Ansprüche 1 bis 10 handelt.

12. Pharmazeutisches Mittel, **dadurch gekennzeichnet**, daß es in einem zur enteralen, parenteralen, topischen oder okularen Verabreichung geeigneten Träger mindestens eine Verbindung der Formel (I) nach einem der Ansprüche 1 bis 10 enthält.

13. Mittel nach Anspruch 12, **dadurch gekennzeichnet**, daß es in geeigneter Form zur topischen oder okularen Verabreichung vorliegt und 0,0001 bis ca. 5 Gew.-% und vorzugsweise 0,001 bis 1 Gew.-% einer Verbindung der Formel (I) enthält.

14. Verwendung einer Verbindung nach einem der Ansprüche 1 bis 10 zur Herstellung eines zur Behandlung dermatologischer, respiratorischer sowie ophthalmologischer Affektionen bestimmten pharmazeutischen Mittels.

15. Kosmetisches Mittel zur Hygiene des Körpers und der Haare, **dadurch gekennzeichnet**, daß es in einem geeigneten kosmetischen Träger mindestens eine Verbindung der Formel (I) nach einem der Ansprüche 1 bis 10 enthält.

16. Kosmetisches Mittel nach Anspruch 15, **dadurch gekennzeichnet**, daß es die Verbindung der Formel (I) in einer Konzentration zwischen 0,0001 und 2 Gew.-% und vorzugsweise zwischen 0,001 und 2 Gew.-% enthält.

17. Mittel nach den Ansprüchen 12, 13, 15 und 16, **dadurch gekennzeichnet**, daß es ein Additiv enthält, ausgewählt unter Hydratisierungsmitteln, Antiseborrhöe-Mitteln, Antiakne-Mitteln, Antibiotika, Haarwuchsmitteln, antiinflammatorischen Mitteln, Carotinoiden und Antipsorias-Mitteln.